(19) Europäisches Patentamt / European Patent Office / Office européen des brevets

(11) EP 3 331 998 B1

(12) **EUROPEAN PATENT SPECIFICATION**

(45) Date of publication and mention of the grant of the patent:
**24.06.2020 Bulletin 2020/26**

(21) Application number: **15798180.4**

(22) Date of filing: **07.08.2015**

(51) Int Cl.:
***C12N 9/10*** *(2006.01)* ***C12P 13/12*** *(2006.01)*

(86) International application number:
**PCT/IB2015/001888**

(87) International publication number:
**WO 2017/025766 (16.02.2017 Gazette 2017/07)**

(54) **PROTEIN THIOCARBOXYLATE-DEPENDENT L-METHIONINE PRODUCTION BY FERMENTATION**

PROTEIN THIOCARBOXYLAT-ABHÄNGIGE FERMENTATIVE HERSTELLUNG VON L-METHIONIN

PRODUCTION FERMENTATIVE DE L-MÉTHIONINE DÉPENDANT DE PROTÉINE COMPRENANT UN GROUPE THIOCARBOXYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.06.2018 Bulletin 2018/24**

(73) Proprietor: **Evonik Operations GmbH**
**45128 Essen (DE)**

(72) Inventors:
- **SOUCAILLE, Philippe**
  **31450 Deyme (FR)**
- **VASSEUR, Perrine**
  **63720 Martres sur Morges (FR)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
**WO-A1-2008/127240 US-A1- 2009 253 186**

- **KALYANARAMAN KRISHNAMOORTHY ET AL: "Protein Thiocarboxylate-Dependent Methionine Biosynthesis in Wolinella succinogenes", JOURNAL OF THE AMERICAN CHEMICAL SOCIETY, vol. 133, no. 2, 19 January 2011 (2011-01-19), pages 379-386, XP055245576, US ISSN: 0002-7863, DOI: 10.1021/ja107424t cited in the application**
- **BOURHY P ET AL: "HOMOSERINE O-ACETYLTRANSFERASE, INVOLVED IN THE LEPTOSPIRA MEYERI METHIONINE BIOSYNTHETIC PATHWAY, IS NOT FEEDBACK INHIBITED", JOURNAL OF BACTERIOLOGY, AMERICAN SOCIETY FOR MICROBIOLOGY, US, vol. 179, no. 13, 1 July 1997 (1997-07-01) , pages 4396-4398, XP002934097, ISSN: 0021-9193**
- **LEE H-S ET AL: "Methionine biosynthesis and its regulation in Corynebacterium glutamicum: Parallel pathways of transsulfuration and direct sulfhydrylation", APPLIED MICROBIOLOGY AND BIOTECHNOLOGY, SPRINGER, DE, vol. 62, no. 5-6, 1 October 2003 (2003-10-01), pages 459-467, XP002355864, ISSN: 0175-7598, DOI: 10.1007/S00253-003-1306-7**
- **M. P. FERLA ET AL: "Bacterial methionine biosynthesis", MICROBIOLOGY, vol. 160, no. Pt_8, 17 June 2014 (2014-06-17), pages 1571-1584, XP055252744, GB ISSN: 1350-0872, DOI: 10.1099/mic.0.077826-0**

EP 3 331 998 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• TIMOTHY H. TRAN ET AL: "A novel mechanism of sulfur transfer catalyzed by O -acetylhomoserine sulfhydrylase in the methionine-biosynthetic pathway of Wolinella succinogenes", ACTA CRYSTALLOGRAPHICA SECTION D: BIOLOGICAL CRYSTALLOGRAPHY., vol. 71, no. 10, 8 September 2011 (2011-09-08), pages 1125-838, XP055252756, DK ISSN: 0907-4449, DOI: 10.1107/S0907444911028010

SEQUENCE LISTING

<110> METABOLIC EXPLORER

<120> PROTEIN THIOCARBOXYLATE-DEPENDENT L-METHIONINE PRODUCTION BY FERMENTATION

<130> B368291D35196

<160> 16

<170> PatentIn version 3.5

<210> 1
<211> 210
<212> DNA
<213> Wolinella succinogenes

<400> 1

**atgaatctca tcatcaacgg agagaataag agttttgaaa aagagggctt aagcgtgaag 60**

**gagcttttgg ttttagaatc ggttaaaatg cctgaaatgg tctccattca gctcaatgac 120**

**gagtttttaa gagagcctga gtatgcgacc acttcgctta aagagggcga tacgattaac 180**

**tttttatatt tcatgggagg gggcgcatga 210**

<210> 2
<211> 69
<212> PRT
<213> Wolinella succinogenes

<400> 2

```
Met Asn Leu Ile Ile Asn Gly Glu Asn Lys Ser Phe Glu Lys Glu Gly
1               5                   10                  15

Leu Ser Val Lys Glu Leu Leu Val Leu Glu Ser Val Lys Met Pro Glu
            20                  25                  30

Met Val Ser Ile Gln Leu Asn Asp Glu Phe Leu Arg Glu Pro Glu Tyr
        35                  40                  45

Ala Thr Thr Ser Leu Lys Glu Gly Asp Thr Ile Asn Phe Leu Tyr Phe
    50                  55                  60

Met Gly Gly Gly Ala
65
```

<210> 3
<211> 393
<212> DNA
<213> Wolinella succinogenes

<400> 3

```
atgctcaaaa tccctaaagc gctctttgat tccattatcg agcacgccca aagagagctt      60

cccttagagg cgtgcggcta tgtagctgga gtggagggcg aggtgaagcg gctctttcct     120

atgaggaatg tcgatgcgag ccctgagcat ttcagctttg atcccgccga acaatttagc     180

gcttttaaag aggcgcaaaa agagggattg cggctcattg gctgctatca ctctcaccca     240

agcactcctg caagaccctc cgatgaggat attcgcctag cctatgacag tagcctcagc     300

tacctcattg tctcgctggc caaggagcct gttttaaact cttttaaaat caaagaggga     360

gtcgtcactc ccgaaaatat cgaggtgatc taa                                  393
```

<210> 4
<211> 130
<212> PRT
<213> Wolinella succinogenes

<400> 4

```
Met Leu Lys Ile Pro Lys Ala Leu Phe Asp Ser Ile Ile Glu His Ala
1               5                   10                  15

Gln Arg Glu Leu Pro Leu Glu Ala Cys Gly Tyr Val Ala Gly Val Glu
            20                  25                  30

Gly Glu Val Lys Arg Leu Phe Pro Met Arg Asn Val Asp Ala Ser Pro
        35                  40                  45

Glu His Phe Ser Phe Asp Pro Ala Glu Gln Phe Ser Ala Phe Lys Glu
    50                  55                  60

Ala Gln Lys Glu Gly Leu Arg Leu Ile Gly Cys Tyr His Ser His Pro
65                  70                  75                  80

Ser Thr Pro Ala Arg Pro Ser Asp Glu Asp Ile Arg Leu Ala Tyr Asp
                85                  90                  95

Ser Ser Leu Ser Tyr Leu Ile Val Ser Leu Ala Lys Glu Pro Val Leu
            100                 105                 110

Asn Ser Phe Lys Ile Lys Glu Gly Val Val Thr Pro Glu Asn Ile Glu
        115                 120                 125

Val Ile
    130
```

<210> 5
<211> 819
<212> DNA
<213> Wolinella succinogenes

<400> 5

```
atgagagagt ttagcgaaga ggagctagaa cgctactcaa ggcatatcat ccttgaagag        60
```

```
gtgggaatcg aggggcaaga gaagatcatg aactccaaag tccttatcat cggtgcaggc      120
ggacttggtt cgcccattgc cttctatttg gccgcagcgg gagtgggaga gatcggaatc      180
atcgatgggg atgtggtgga tcggagcaat cttcagcgcc agatcattca caccacggat      240
gagattggaa tccccaaagt cgaatcggca cgccgcaagc tcaaggcgct caatcccaat      300
attcgagttc aaacttggca gatcatgatc aatgccgaaa acatctcacg aatcatcgcc      360
ccctacgatt ttatcatcga tgggacggat aattttgcgg ccaaattcct catcaatgat      420
gcgtgtgtca tggcgggcaa accctactcc cacggtggaa tcttgaaatt tgcagggcag      480
agcatgacca taaaaccggg agagagcgcc tgctatgcgt gtgtctttga tcagcctccc      540
cccgctggct ctattcccac ctgctctagc gcagggattt taggggcgat tgcagggatg      600
cttggcacca ttcaagccgc cgaggcgctc aaagtgatta caggcgtagg cgaacccctc      660
tataatcgcc ttttaagctt tgatgccaaa agcatgaatt tccgcaccgt gaaattcacc      720
aaaaacccccc attgtcgtgt ttgcggaggc gagggagtga agattttacg cgaatatgaa      780
caacccatat gcgaggtgaa tcatgctcaa aatccctaa                             819
```

<210> 6
<211> 272
<212> PRT
<213> Wolinella succinogenes

<400> 6

```
Met Arg Glu Phe Ser Glu Glu Glu Leu Glu Arg Tyr Ser Arg His Ile
1               5                   10                  15

Ile Leu Glu Glu Val Gly Ile Glu Gly Gln Glu Lys Ile Met Asn Ser
            20                  25                  30

Lys Val Leu Ile Ile Gly Ala Gly Gly Leu Gly Ser Pro Ile Ala Phe
        35                  40                  45

Tyr Leu Ala Ala Ala Gly Val Gly Glu Ile Gly Ile Ile Asp Gly Asp
    50                  55                  60

Val Val Asp Arg Ser Asn Leu Gln Arg Gln Ile Ile His Thr Thr Asp
65                  70                  75                  80

Glu Ile Gly Ile Pro Lys Val Glu Ser Ala Arg Arg Lys Leu Lys Ala
                85                  90                  95

Leu Asn Pro Asn Ile Arg Val Gln Thr Trp Gln Ile Met Ile Asn Ala
            100                 105                 110
```

Glu Asn Ile Ser Arg Ile Ile Ala Pro Tyr Asp Phe Ile Ile Asp Gly
115 120 125

Thr Asp Asn Phe Ala Ala Lys Phe Leu Ile Asn Asp Ala Cys Val Met
130 135 140

Ala Gly Lys Pro Tyr Ser His Gly Gly Ile Leu Lys Phe Ala Gly Gln
145 150 155 160

Ser Met Thr Ile Lys Pro Gly Glu Ser Ala Cys Tyr Ala Cys Val Phe
165 170 175

Asp Gln Pro Pro Pro Ala Gly Ser Ile Pro Thr Cys Ser Ser Ala Gly
180 185 190

Ile Leu Gly Ala Ile Ala Gly Met Leu Gly Thr Ile Gln Ala Ala Glu
195 200 205

Ala Leu Lys Val Ile Thr Gly Val Gly Glu Pro Leu Tyr Asn Arg Leu
210 215 220

Leu Ser Phe Asp Ala Lys Ser Met Asn Phe Arg Thr Val Lys Phe Thr
225 230 235 240

Lys Asn Pro His Cys Arg Val Cys Gly Gly Glu Gly Val Lys Ile Leu
245 250 255

Arg Glu Tyr Glu Gln Pro Ile Cys Glu Val Asn His Ala Gln Asn Pro
260 265 270

<210> 7
<211> 764
<212> PRT
<213> Wolinella succinogenes

<400> 7

Met Ser His Tyr Thr Leu Pro Pro Ser Val Ala Glu Asp Phe Ala Ser
1 5 10 15

Phe Lys Glu Ser His Ala Asp Phe Met Ala Gly Lys Leu Asp Ala Leu
20 25 30

Thr Phe Lys Thr Ile Arg Val Pro Phe Gly Ile Tyr Glu Gln Arg Glu
35 40 45

Ser Asp Thr Tyr Met Val Arg Val Lys Leu Ala Gly Gly Ile Leu Thr
50 55 60

```
Pro Ala Gln Leu His Ser Leu Ala Leu Leu Ala Glu His Tyr Ala Lys
65                  70                  75                  80

Pro His Leu His Val Thr Thr Arg Gly Gly Val Gln Leu His Tyr Ala
                85                  90                  95

Lys Leu Gln Asp Leu Pro Gln Ile Ile Gln Ala Leu His Glu Met Gly
            100                 105                 110

Leu Thr Gly Arg Gly Gly Gly Gly Asn Thr Val Arg Asn Ile Thr Ala
        115                 120                 125

Asp Pro Tyr Ala Gly Ile Ala Pro Asp Glu Ala Phe Asp Val Thr Pro
    130                 135                 140

Cys Ala Leu Ser Leu Thr Thr Lys Met Leu Glu Thr Lys Asp Ser Tyr
145                 150                 155                 160

Ser Leu Pro Arg Lys Phe Lys Ile Ala Phe Ser Gly Ser Ser Glu Asp
                165                 170                 175

Arg Gly Gly Ala Thr Tyr Ile Asp Val Gly Phe Ile Ala Lys Ile His
            180                 185                 190

Glu Gly Val Arg Gly Phe Arg Leu Phe Val Ala Gly Gly Met Gly Ala
        195                 200                 205

Lys Ser Arg Leu Gly Ser Ala Phe Ile Asp Phe Leu Pro Gln Glu Glu
    210                 215                 220

Ile Phe Leu Phe Ser Gln Ala Ile Lys Gln Val Phe Asp Gln His Gly
225                 230                 235                 240

Asn Arg Lys Asn Lys His Ala Ala Arg Leu Arg Phe Leu Ile Glu Glu
                245                 250                 255

Leu Gly Glu Gly Gln Phe Arg Glu Leu Val Phe Lys Glu Val Gln Ala
            260                 265                 270

Leu Arg Asp Gln Gly Gly Trp Glu Ile Lys Leu Glu Glu Ala Phe Glu
        275                 280                 285

Ala Ile Pro Leu Glu Ser Asp Glu Ile Pro Pro Leu Asn Gln Glu Gln
    290                 295                 300

Lys Leu Trp Trp Asn Arg Phe Val Ile Pro Gln Lys Gln Lys Gly Tyr
305                 310                 315                 320
```

```
Tyr Ser Ala Lys Val Pro Leu Lys Leu Gly Asp Leu Glu Ser Glu His
                325                 330                 335

Ala Lys Ser Leu Ala Gln Ala Leu Gln Gly Phe Lys Tyr Gly Lys Glu
            340                 345                 350

Ser Ile Arg Phe Gly Ser Asp Gln Asn Leu Tyr Leu Arg Asn Leu Gln
        355                 360                 365

Ala Asp Glu Leu Leu Ser Leu Tyr Pro Leu Ile Gln Glu Leu Ser Gly
    370                 375                 380

Gln Ser Ser Arg Ala Arg Ile Leu Gly Asp Met Val Ala Cys Thr Gly
385                 390                 395                 400

Ala Ala Thr Cys Gln Leu Gly Ile Thr Arg Pro Arg Gly Ala Val Val
                405                 410                 415

Ala Ile Glu Lys Glu Leu Gln Lys Ala Asn Ile Asp Leu Asp Ala Leu
            420                 425                 430

Gln Gly Phe Arg Ile His Leu Ser Gly Cys Pro Asn Ser Cys Gly Lys
        435                 440                 445

His Ala Ile Ala Asp Leu Gly Phe Phe Gly Lys Val Asn Arg Glu Gly
    450                 455                 460

Gly His Pro Tyr Pro Ala Tyr Asn Val Leu Val Gly Ala Ile Ile Gln
465                 470                 475                 480

Glu Asp Ser Thr Arg Phe Ala Lys Lys Ile Ala Glu Val Ser Ala Tyr
                485                 490                 495

Ala Leu Pro Leu Phe Val Thr Glu Val Leu Lys Leu Trp Leu His Ala
            500                 505                 510

Lys Pro His Tyr Ala Asn Phe Ala Ala Trp Val Asp Gln Glu Gly Glu
        515                 520                 525

Ala Gln Ile Ile His Leu Ala Ser Ser Tyr Ala Lys Ile Pro Ser Phe
    530                 535                 540

Glu Glu Asp Lys Asn Pro Tyr Phe Asp Tyr Gly Ser Glu Glu Ile Phe
545                 550                 555                 560

Ser Leu Lys Gly Arg Gly Val Gly Glu Cys Ser Ala Gly Met Tyr Asp
                565                 570                 575
```

```
Leu Ile Glu Ala Asp Lys Lys Ala Leu Lys Glu Ala Leu Glu Gly Gly
            580                 585                 590

Asp Glu Glu Glu Asn Leu Ala Lys Ile Arg Leu Leu Ala Ser Arg Met
        595                 600                 605

Leu Leu Ile Thr Lys Gly Glu Glu Ala Arg Asp Glu Arg Ser Val Leu
    610                 615                 620

Gln Ala Phe Arg Arg Leu Phe Val Glu Gly Gly Leu Ile Asp Ser Ser
625                 630                 635                 640

Phe Ala Pro Leu Leu Glu Gly Arg Pro Arg Ile Glu Leu Lys Ala Leu
                645                 650                 655

Gly Glu Ala Val Ile Ala Leu Tyr Gly Thr Met Asp Asn Ser Leu Lys
            660                 665                 670

Phe Ala Lys Glu Gln Pro Lys Glu Ala Pro Leu Ala Pro Thr Ser Ser
        675                 680                 685

Ser Ser Ser Ala His Arg Phe Lys Asp Tyr Gln Gly Val Ala Cys Pro
    690                 695                 700

Met Asn Phe Val Lys Thr Lys Met Asp Leu Ala Gln Met Gln Ser Gly
705                 710                 715                 720

Glu Ile Leu Glu Ile Leu Leu Asp Glu Gly Ala Pro Ile Glu Asn Val
                725                 730                 735

Pro Lys Ser Val Ala Asn Glu Gly His Leu Ile Leu Gly Gln Thr Lys
            740                 745                 750

Glu Gly Lys Gly Trp Arg Val Arg Ile Gln Lys Arg
        755                 760
```

<210> 8
<211> 2295
<212> DNA
<213> Wolinella succinogenes

<400> 8

```
atgagccact acaccctacc cccctccgtc gctgaggatt ttgcctcttt caaagagagt      60
cacgccgact ttatggcggg caaactcgat gcactgacct ttaaaaccat tcgtgttccc     120
tttggaatct atgagcaacg agagagcgac acctatatgg tgagagtgaa gctagcaggg     180
ggcattctta ctcccgctca actccactcg ctcgcccttc tagccgagca ttacgccaaa     240
```

```
ccccatctcc atgtcaccac gcgcggaggc gtccagctcc actatgccaa gctccaagat      300
ctccccccaaa tcatccaagc gcttcatgag atggggctca cagggcgagg cggaggcggg      360
aacaccgtgc gcaacatcac tgccgatccc tatgcaggaa tcgcccctga tgaggcattt      420
gatgtcactc cttgtgcgct ctctctcacc acgaaaatgc ttgagacaaa agactcctat      480
tcgctcccaa gaaaattcaa aatcgccttc agtggctcta gcgaggatag gggcggagcc      540
acctacatcg atgtgggatt catcgccaag atccacgaag gagtgcgagg attccgtcta      600
tttgtcgctg gggcatggg ggctaaatca cgccttggaa gcgcttttat cgacttttta      660
ccccaagaag agatattcct tttttctcaa gccatcaagc aggtttttga ccagcacggc      720
aatcgcaaaa acaagcacgc cgcaagactt cgattcctca tcgaggagct tggagagggg      780
caatttagag agcttgtctt taaagaggtc caagcgcttc gcgatcaagg ggggtgggag      840
attaagctag aggaggcttt tgaggcaatc cctttagaga gcgatgagat tcctccatta      900
aatcaagagc aaaaactctg gtggaatcgc tttgttatcc ctcaaaaaca aaaaggctac      960
tacagtgcca aagtcccccct gaaattgggc gacttggaat cagaacacgc caaatccctt     1020
gcccaagccc ttcaaggatt caagtatggc aaagagtcga ttcgttttgg aagcgatcag     1080
aatctctacc taagaaacct ccaagccgat gaacttctct cgctctaccc ccttattcaa     1140
gagctctcag gacaatcaag ccgcgcaagg attcttgggg atatggtcgc ttgcacaggc     1200
gcggccactt gccagcttgg aatcacgcgc cccagaggag ccgtggtggc aatagaaaaa     1260
gagctccaaa aagccaatat cgacctagat gcgcttcaag gctttagaat ccatctctct     1320
ggatgcccca atagctgcgg aaaacacgcc atcgctgatt tgggattttt tggcaaagtg     1380
aatcgagagg gaggtcatcc ctatcccgcc tataatgtcc ttgtgggcgc catcatccaa     1440
gaagattcca ctcgatttgc caaaaaaatc gccgaggtga gcgcctatgc tcttcctctt     1500
tttgtgacag aggtgctcaa gctttggctc catgccaagc cccattatgc gaactttgct     1560
gcatgggtgg atcaagaggg cgaggctcaa atcatccacc tcgcctcttc ctatgccaag     1620
attcctagct ttgaagagga taaaaacccc tactttgact acggaagcga agagatattt     1680
tcgctcaaag gtcgaggagt gggtgagtgc agcgcgggaa tgtatgacct aattgaggcg     1740
gataaaaaag cactcaaaga ggcgctagag ggaggagacg aggaggagaa tctcgccaaa     1800
atccgcctgc tcgcctcaag aatgctcctc atcaccaaag gcgaagaggc gcgcgatgaa     1860
cgctcggtgc ttcaggcgtt caggcgactt tttgtcgaag gaggattgat tgattcctct     1920
tttgcgcccc ttttagaagg caggccaaga atcgagctca aagctctagg tgaggcggtc     1980
attgctcttt atggcaccat ggataatagc ctgaaatttg ccaaagagca gccaaaagaa     2040
gcacctcttg cccccacctc tagctcctct agtgcccatc gctttaaaga ttaccaaggg     2100
```

gtcgcctgcc ccatgaactt tgtcaagacc aaaatggact tagcccaaat gcaaagcggc 2160

gagattttgg agattttgct cgatgagggc gctcccatcg aaaatgtccc caaatccgta 2220

gccaacgagg gtcacctcat cctaggccaa accaaagagg gaaaagggtg gcgcgtgagg 2280

attcaaaaac gatga 2295

<210> 9
<211> 1140
<212> DNA
<213> Leptospira meyeri

<400> 9

atgcctacct ccgaacagaa cgagttttcc cacggatccg taggtgtcgt atatactcag 60

agcattcgat ttgagtcttt gactctagag gggggtgaaa ccatcactcc tcttgaaatt 120

gcctacgaaa cgtatggcac tctcaatgaa aaaaaagaca atgccattct agtttgccat 180

gcgctttcgg gagatgctca tgcagcaggt ttccatgaag gagacaaacg tcctggctgg 240

tgggattatt atattggacc gggcaaatcc tttgatacca atcgttactt tatcatttct 300

tccaacgtaa ttggtggttg taagggttcc agtggaccac ttaccatcaa tgggaaaaat 360

ggaaaaccat tccaatccac ttttcccttt gtctccatag gagatatggt gaatgctcaa 420

gaaaaattaa tcagccattt tggaattcat aaactatttg ctgttgccgg tggttcgatg 480

ggtggaatgc aagccttaca atggtcagtc gcatacccag atcggctcaa aaattgtatc 540

gtgatggcat cttcttccga acattctgca caacaaattg cctttaatga agtgggaaga 600

caagccattc tttctgatcc caattggaac caaggtttgt acacccagga aaacagaccg 660

tcaaagggac ttgctcttgc tcgaatgatg ggtcatatca cttacttaag cgatgaaatg 720

atgagagaaa aatttggtcg taaaccaccc aaaggaaata tccaatccac agactttgcg 780

gtaggaagtt atctaatcta ccaaggcgaa tcctttgtcg atcggtttga tgcaaactca 840

tatatttatg ttacaaaagc attggatcat tttagtttag gtacaggaaa agaacttaca 900

aaggtattgg caaaagtgag atgccggttt ttggtagtgg cttatacttc cgattggttg 960

tatccaccgt atcaatctga agaaattgtg aaatctttgg aagtgaatgc tgttcccgtt 1020

agttttgtag aactcaacaa tccagcagga cgacatgata gtttttgtt accaagtgag 1080

caacaagact cgatcctaag agatttttta agttctacgg acgaaggagt tttcctttga 1140

<210> 10
<211> 378
<212> PRT
<213> Leptospira meyeri

<400> 10

Met Pro Thr Ser Glu Gln Asn Glu Phe Ser His Gly Ser Val Gly Val
1               5                   10                  15

```
Val Tyr Thr Gln Ser Ile Arg Phe Glu Ser Leu Thr Leu Glu Gly Gly
            20                  25                  30

Glu Thr Ile Thr Pro Leu Glu Ile Ala Tyr Glu Thr Tyr Gly Thr Leu
        35                  40                  45

Asn Glu Lys Lys Asp Asn Ala Ile Leu Val Cys His Ala Leu Ser Gly
    50                  55                  60

Asp Ala His Ala Ala Gly Phe His Glu Gly Asp Lys Arg Pro Gly Trp
65                  70                  75                  80

Trp Asp Tyr Tyr Ile Gly Pro Gly Lys Ser Phe Asp Thr Asn Arg Tyr
                85                  90                  95

Phe Ile Ile Ser Ser Asn Val Ile Gly Gly Cys Lys Gly Ser Ser Gly
            100                 105                 110

Pro Leu Thr Ile Asn Gly Lys Asn Gly Lys Pro Phe Gln Ser Thr Phe
        115                 120                 125

Pro Phe Val Ser Ile Gly Asp Met Val Asn Ala Gln Glu Lys Leu Ile
    130                 135                 140

Ser His Phe Gly Ile His Lys Leu Phe Ala Val Ala Gly Gly Ser Met
145                 150                 155                 160

Gly Gly Met Gln Ala Leu Gln Trp Ser Val Ala Tyr Pro Asp Arg Leu
                165                 170                 175

Lys Asn Cys Ile Val Met Ala Ser Ser Ser Glu His Ser Ala Gln Gln
            180                 185                 190

Ile Ala Phe Asn Glu Val Gly Arg Gln Ala Ile Leu Ser Asp Pro Asn
        195                 200                 205

Trp Asn Gln Gly Leu Tyr Thr Gln Glu Asn Arg Pro Ser Lys Gly Leu
    210                 215                 220

Ala Leu Ala Arg Met Met Gly His Ile Thr Tyr Leu Ser Asp Glu Met
225                 230                 235                 240

Met Arg Glu Lys Phe Gly Arg Lys Pro Pro Lys Gly Asn Ile Gln Ser
                245                 250                 255

Thr Asp Phe Ala Val Gly Ser Tyr Leu Ile Tyr Gln Gly Glu Ser Phe
```

```
                260                 265                 270

        Val Asp Arg Phe Asp Ala Asn Ser Tyr Ile Tyr Val Thr Lys Ala Leu
                275                 280                 285

        Asp His Phe Ser Leu Gly Thr Gly Lys Glu Leu Thr Lys Val Leu Ala
            290                 295                 300

        Lys Val Arg Cys Arg Phe Leu Val Val Ala Tyr Thr Ser Asp Trp Leu
        305                 310                 315                 320

        Tyr Pro Pro Tyr Gln Ser Glu Glu Ile Val Lys Ser Leu Glu Val Asn
                        325                 330                 335

        Ala Val Pro Val Ser Phe Val Glu Leu Asn Asn Pro Ala Gly His Asp
                    340                 345                 350

        Ser Phe Leu Leu Pro Ser Glu Gln Gln Asp Ser Ile Leu Arg Asp Phe
                355                 360                 365

        Leu Ser Ser Thr Asp Glu Gly Val Phe Leu
            370                 375
```

<210> 11
<211> 1224
<212> DNA
<213> Wolinella succinogenes

<400> 11

```
gtgaggggat tcaccacgag ggcgcttcat gttcccaagg ccaagagaga cgtccatgga        60
gcgcttcgca cgcccgtcta tgataacgcg gcgtttgagt ttgaaaatag cgatgagatc       120
gcccaagttt ccttgggtag ggcacttggg catgtctata gccgctctag caaccccacg       180
gtggaggatt tggagcagcg cctcaagaat ctcacgggag ccttgggggt gttggcgcta       240
gggagtggga tggcagcgat ctcaacggcg attttgacct tggcgagggc aggggatagt       300
gtggtcacca ccgatcgtct ctttgggcac accctctcgc tctttcaaaa gacactgcct       360
agctttggaa tcgaggttcg ttttgtggat gtgatggatt ctctagcagt ggagcatgcc       420
tgtgatgaga caaccaagct tcttttcttg gagaccatca gcaatcctca acttcaagtg       480
gccgatctag aggctctctc taaagtggtg cacgccaaag gaatcccact agtggtcgat       540
acgaccatga cccctcccta tcttttggag gcaaaacgct taggggtgga catcgaagtg       600
ctctcttcaa ccaaattcat ctcaggcgga ggaacaagcg tgggaggagt cttgattgat       660
catggacttt ttgagtggaa gagtctccct tcgctcgctc cctattatgc caaggcgggg       720
ccgatggctt tcctctacaa ggcgcgcaag gaggtgtttc aaaatctagg accctcgctt       780
agcccccaca acgcctatct tcaaagtttg ggttggaga cgatggcgct tcgaatcgag        840
cgttcgtgtc aaaacgccca agagcttgcg cattggcttt tgtctatccc tcaggtgaaa       900
tgcgtcaatc acccttcgct ccctgattct ccttttatg cgattgctaa gcgtcagttt        960
cgctacgcag gctcgattct cacctttgag ttggagagca aggaggcctc ctatcgcttc      1020
atggatgcgc tcaagctcat tcggcgcgcc accaatatcc atgacaataa aagcctcatc      1080
ctctccccct atcatgtcat ctatgccctc aatagccacg aagagcgctt aaagcttgag      1140
atatctcctg caatgatgag gctttctgtg ggaattgaag agattgaaga tctgaaagag      1200
gatattttgc aagcgctatg ttaa                                             1224
```

<210> 12
<211> 407
<212> PRT
<213> Wolinella succinogenes

<400> 12

```
Val Arg Gly Phe Thr Thr Arg Ala Leu His Val Pro Lys Ala Lys Arg
1               5                   10                  15

Asp Val His Gly Ala Leu Arg Thr Pro Val Tyr Asp Asn Ala Ala Phe
            20                  25                  30

Glu Phe Glu Asn Ser Asp Glu Ile Ala Gln Val Ser Leu Gly Arg Ala
        35                  40                  45

Leu Gly His Val Tyr Ser Arg Ser Ser Asn Pro Thr Val Glu Asp Leu
    50                  55                  60

Glu Gln Arg Leu Lys Asn Leu Thr Gly Ala Leu Gly Val Leu Ala Leu
65                  70                  75                  80

Gly Ser Gly Met Ala Ala Ile Ser Thr Ala Ile Leu Thr Leu Ala Arg
                85                  90                  95

Ala Gly Asp Ser Val Val Thr Thr Asp Arg Leu Phe Gly His Thr Leu
            100                 105                 110

Ser Leu Phe Gln Lys Thr Leu Pro Ser Phe Gly Ile Glu Val Arg Phe
        115                 120                 125

Val Asp Val Met Asp Ser Leu Ala Val Glu His Ala Cys Asp Glu Thr
    130                 135                 140

Thr Lys Leu Leu Phe Leu Glu Thr Ile Ser Asn Pro Gln Leu Gln Val
145                 150                 155                 160
```

```
Ala Asp Leu Glu Ala Leu Ser Lys Val Val His Ala Lys Gly Ile Pro
                165                 170                 175

Leu Val Val Asp Thr Thr Met Thr Pro Pro Tyr Leu Leu Glu Ala Lys
            180                 185                 190

Arg Leu Gly Val Asp Ile Glu Val Leu Ser Ser Thr Lys Phe Ile Ser
        195                 200                 205

Gly Gly Gly Thr Ser Val Gly Gly Val Leu Ile Asp His Gly Leu Phe
    210                 215                 220

Glu Trp Lys Ser Leu Pro Ser Leu Ala Pro Tyr Tyr Ala Lys Ala Gly
225                 230                 235                 240

Pro Met Ala Phe Leu Tyr Lys Ala Arg Lys Glu Val Phe Gln Asn Leu
                245                 250                 255

Gly Pro Ser Leu Ser Pro His Asn Ala Tyr Leu Gln Ser Leu Gly Leu
            260                 265                 270

Glu Thr Met Ala Leu Arg Ile Glu Arg Ser Cys Gln Asn Ala Gln Glu
        275                 280                 285

Leu Ala His Trp Leu Leu Ser Ile Pro Gln Val Lys Cys Val Asn His
    290                 295                 300

Pro Ser Leu Pro Asp Ser Pro Phe Tyr Ala Ile Ala Lys Arg Gln Phe
305                 310                 315                 320

Arg Tyr Ala Gly Ser Ile Leu Thr Phe Glu Leu Glu Ser Lys Glu Ala
                325                 330                 335

Ser Tyr Arg Phe Met Asp Ala Leu Lys Leu Ile Arg Arg Ala Thr Asn
            340                 345                 350

Ile His Asp Asn Lys Ser Leu Ile Leu Ser Pro Tyr His Val Ile Tyr
        355                 360                 365

Ala Leu Asn Ser His Glu Glu Arg Leu Lys Leu Glu Ile Ser Pro Ala
    370                 375                 380

Met Met Arg Leu Ser Val Gly Ile Glu Glu Ile Glu Asp Leu Lys Glu
385                 390                 395                 400

Asp Ile Leu Gln Ala Leu Cys
                405
```

<210> 13
<211> 1224
<212> DNA
<213> Wolinella succinogenes

<400> 13

```
atgaggggat tcaccacgag ggcgcttcat gttcccaagg ccaagagaga cgtccatgga      60
gcgcttcgca cgcccgtcta tgataacgcg gcgtttgagt ttgaaaatag cgatgagatc     120
gcccaagttt ccttgggtag ggcacttggg catgtctata gccgctctag caaccccacg     180
gtggaggatt tggagcagcg cctcaagaat ctcacgggag ccttgggggt gttggcgcta     240
gggagtggga tggcagcgat ctcaacggcg attttgacct tggcgagggc aggggatagt     300
gtggtcacca ccgatcgtct ctttgggcac accctctcgc tctttcaaaa gacactgcct     360
agctttggaa tcgaggttcg ttttgtggat gtgatggatt ctctagcagt ggagcatgcc     420
tgtgatgaga caaccaagct tcttttcttg gagaccatca gcaatcctca acttcaagtg     480
gccgatctag aggctctctc taaagtggtg cacgccaaag gaatcccact agtggtcgat     540
acgaccatga cccctcccta tcttttggag gcaaaacgct taggggtgga catcgaagtg     600
ctctcttcaa ccaaattcat ctcaggcgga ggaacaagcg tgggaggagt cttgattgat     660
catggacttt ttgagtggaa gagtctccct tcgctcgctc cctattatgc caaggcgggg     720
ccgatggctt tcctctacaa ggcgcgcaag gaggtgtttc aaaatctagg accctcgctt     780
agcccccaca acgcctatct tcaaagtttg gggttggaga cgatggcgct tcgaatcgag     840
cgttcgtgtc aaaacgccca agagcttgcg cattggcttt tgtctatccc tcaggtgaaa     900
tgcgtcaatc acccttcgct ccctgattct cctttttatg cgattgctaa gcgtcagttt     960
cgctacgcag gctcgattct cacctttgag ttggagagca aggaggcctc ctatcgcttc    1020
atggatgcgc tcaagctcat tcggcgcgcc accaatatcc atgacaataa aagcctcatc    1080
ctctcccccct atcatgtcat ctatgccctc aatagccacg aagagcgctt aaagcttgag   1140
atatctcctg caatgatgag gctttctgtg ggaattgaag agattgaaga tctgaaagag    1200
gatattttgc aagcgctatg ttaa                                           1224
```

<210> 14
<211> 407
<212> PRT
<213> Wolinella succinogenes

<400> 14

Met Arg Gly Phe Thr Thr Arg Ala Leu His Val Pro Lys Ala Lys Arg
1               5                   10                  15

Asp Val His Gly Ala Leu Arg Thr Pro Val Tyr Asp Asn Ala Ala Phe

20 25 30

Glu Phe Glu Asn Ser Asp Glu Ile Ala Gln Val Ser Leu Gly Arg Ala
35 40 45

Leu Gly His Val Tyr Ser Arg Ser Ser Asn Pro Thr Val Glu Asp Leu
50 55 60

Glu Gln Arg Leu Lys Asn Leu Thr Gly Ala Leu Gly Val Leu Ala Leu
65 70 75 80

Gly Ser Gly Met Ala Ala Ile Ser Thr Ala Ile Leu Thr Leu Ala Arg
85 90 95

Ala Gly Asp Ser Val Val Thr Thr Asp Arg Leu Phe Gly His Thr Leu
100 105 110

Ser Leu Phe Gln Lys Thr Leu Pro Ser Phe Gly Ile Glu Val Arg Phe
115 120 125

Val Asp Val Met Asp Ser Leu Ala Val Glu His Ala Cys Asp Glu Thr
130 135 140

Thr Lys Leu Leu Phe Leu Glu Thr Ile Ser Asn Pro Gln Leu Gln Val
145 150 155 160

Ala Asp Leu Glu Ala Leu Ser Lys Val Val His Ala Lys Gly Ile Pro
165 170 175

Leu Val Val Asp Thr Thr Met Thr Pro Pro Tyr Leu Leu Glu Ala Lys
180 185 190

Arg Leu Gly Val Asp Ile Glu Val Leu Ser Ser Thr Lys Phe Ile Ser
195 200 205

Gly Gly Gly Thr Ser Val Gly Gly Val Leu Ile Asp His Gly Leu Phe
210 215 220

Glu Trp Lys Ser Leu Pro Ser Leu Ala Pro Tyr Tyr Ala Lys Ala Gly
225 230 235 240

Pro Met Ala Phe Leu Tyr Lys Ala Arg Lys Glu Val Phe Gln Asn Leu
245 250 255

Gly Pro Ser Leu Ser Pro His Asn Ala Tyr Leu Gln Ser Leu Gly Leu
260 265 270

```
Glu Thr Met Ala Leu Arg Ile Glu Arg Ser Cys Gln Asn Ala Gln Glu
        275                 280                 285

Leu Ala His Trp Leu Leu Ser Ile Pro Gln Val Lys Cys Val Asn His
    290                 295                 300

Pro Ser Leu Pro Asp Ser Pro Phe Tyr Ala Ile Ala Lys Arg Gln Phe
305                 310                 315                 320

Arg Tyr Ala Gly Ser Ile Leu Thr Phe Glu Leu Glu Ser Lys Glu Ala
                325                 330                 335

Ser Tyr Arg Phe Met Asp Ala Leu Lys Leu Ile Arg Arg Ala Thr Asn
            340                 345                 350

Ile His Asp Asn Lys Ser Leu Ile Leu Ser Pro Tyr His Val Ile Tyr
        355                 360                 365

Ala Leu Asn Ser His Glu Glu Arg Leu Lys Leu Glu Ile Ser Pro Ala
    370                 375                 380

Met Met Arg Leu Ser Val Gly Ile Glu Glu Ile Glu Asp Leu Lys Glu
385                 390                 395                 400

Asp Ile Leu Gln Ala Leu Cys
                405
```

<210> 15
<211> 50
<212> DNA
<213> artificial

<220>
<223> DmetA*11-1 homologous sequence for metA*11 deletion

<400> 15
tgtagtgagg taatcaggtt atgccgattc gtgtgccgga cgagctaccc 50

<210> 16
<211> 50
<212> DNA
<213> artificial

<220>
<223> DmetA*11-2 homologous sequence for metA*11 deletion

<400> 16
tcttctgtga tagtcgatcg ttaagcgatt cagcacctta cctcaggcac 50

## Description

### FIELD OF THE INVENTION

The present invention relates to a recombinant microorganism useful for the production of L-methionine and process for the preparation of L-methionine. The microorganism of the invention is modified in a way that the L-methionine production is improved by using a thiocarboxylated protein as sulfur donor and by overproducing an enzyme having homoserine O-acetyltransferase activity without feedback inhibition by methionine and/or S-adenosylmethionine and an enzyme having O-acetylhomoserine sulfhydrylase activity.

### PRIOR ART

Sulfur-containing compounds such as cysteine, homocysteine, methionine or S-adenosylmethionine are critical to cellular metabolism. In particular L-methionine, an essential amino acid, which cannot be synthesized by animals, plays an important role in many body functions. Most of the methionine produced industrially is widely used as an animal feed and food additive.

With the decreased use of animal-derived proteins as a result of BSE (bovine spongiform encephalopathy) and chicken flu, the demand for pure methionine has increased. Commonly, D,L-methionine is produced chemically from acrolein, methyl mercaptan and hydrogen cyanide. However, the racemic mixture does not perform as well as pure L-methionine (Saunderson, 1985). Additionally, although pure L-methionine can be produced from racemic methionine, for example, through the acylase treatment of N-acetyl-D,L-methionine, this dramatically increases production costs. Accordingly, the increasing demand for pure L-methionine coupled with environmental concerns render microbial production of methionine an attractive prospect.

Other important amino acids, such as lysine, threonine and tryptophan are produced via fermentation for use in animal feed. Therefore, these amino acids can be made using glucose and other renewable resources as starting materials. The production of L-methionine via fermentation has not been successful yet, but the development of the technology is on going.

Different approaches for the optimization of L-methionine production in microorganisms have been described previously (see, for example, patents or patent applications US 7,790,424, US 7,611,873, WO02/10209, WO2005/059093, WO2006/008097, WO2007/0770441, WO2009/043803 and WO2012/098042); however, industrial production of L-methionine from microorganisms requires further improvements.

In these approaches the production of L-methionine is described as using cysteine as sulfur donor, and so the cysteine biosynthetic pathway is optimized by overproducing the different proteins involved in:

(i) The assimilation of sulfate into sulfide, with sequentially the sulfate adenylyltransferase, the adenylylsulfate kinase, the sulfite reductase and the 3'-phospho-adenylylsulfate reductase encoded by the operons *cysDNC* and *cysJIH,*
(ii) The cysteine synthesis, by the serine acetyltransferase and the cysteine synthase encoded by *cysE* and *cysM* genes,
(iii) The transsulfuration that means incorporation of the sulfur from cysteine to succinyl-homoserine giving γ-cysthathionine and then homocysteine via the O-succinylhomoserine (thiol)-lyase and the cystathionine-β-lyase encoded by *metB* and *metC* genes respectively.

However, the optimization of the cysteine biosynthetic pathway necessary for the production of L-methionine is difficult. In fact, the accumulation of cysteine is toxic for the microorganism and so rapidly degraded by cysteinases if it is not used, which means if the cysteine is not produced exactly at the appropriate moment during the L-methionine production process. Moreover, among the known cysteinases present in *E. coli* there is MetC whose production is unavoidable and necessarily increased in the methionine producer strain as MetC is responsible for the conversion of γ-cysthathionine into homocysteine (WO2005/111202) in the methionine biosynthetic pathway.

To by-pass these difficulties, Arkema and CJ Cheiljedang Corporation claim the production of L-methionine in 3 steps: two biosynthesis processes to produce in one hand the methionine precursor, the O-acetyl-L-homoserine or the O-succinyl-L-homoserine, and in another hand the enzyme responsible for the transformation of the precursor in methionine; MetY or MetZ, the O-acetylhomoserine- or the O-succinylhomoserine sulfhydrylases respectively. In a third step, they describe the enzymatic bioconversion of the methionine precursor into L-methionine by MetY or MetZ enzyme in the presence of methyl-mercaptan as sulfur donor (WO2008/013432). Indeed, with this technology it is not necessary to optimize the production of cysteine in the microorganism, as the methyl-mercaptan is the sulfur donor.

Another alternative to the use of cysteine is described by CJ Cheiljedang Corporation and Cargill in patent WO2008/127240 in which they claim microorganisms that produce methionine from exogenous genes coding for homocysteine synthase and so providing a direct sulfhydrylation pathway, which means incorporation of the sulfur directly from sulfide to acetyl-homoserine giving in one step homocysteine.

In the literature it is described that protein thiocarboxylates are members of a growing family of biosynthetic sulfide donors and are involved in a variety of biosynthetic pathways, including vitamin B1 (Taylor et al., 1998) and cysteine (Agren et al., 2008). Recently, a protein thiocarboxylate-dependent methionine biosynthetic pathway was identified in *Wolinella succinogenes* (Krishnamoorthy et al., 2011). In this pathway, (i) the enzymes involved in assimilation of sulfate into sulfide are alike to those of *E. coli;* CysDNC for sulfate adenylyltransferase and adenylylsulfate kinase, CysH for 3'-phospho-adenylylsulfate reductase and Sir for sulfite reductase equivalent to CysJI; (ii) then the carboxy terminal alanine of a novel sulfur transfer protein, HcyS-Ala is removed in a reaction catalysed by a metalloprotease, HcyD, giving HcyS; (iii) HcyF, an ATP-utilizing enzyme, catalyses the adenylation of HcyS; (iv) HcyS acyl-adenylate (HcyS-COOAMP) then undergoes nucleophilic substitution by bisulfide produced by Sir to give the HcyS thiocarboxylate (HcyS-COSH); (v) this adds to O-acetylhomoserine to give HcyS-homocysteine in a PLP-dependent reaction catalysed by MetY (O-acetylhomoserine-sulfhydrylase); (vi) HcyD mediated hydrolysis liberates homocysteine, (vii) a final methylation catalysed by MetE, the homocysteine transmethylase, completes the methionine biosynthesis (cf Fig 1.).

Inventors have found surprisingly that expression of the genes coding for the protein thiocarboxylate dependent methionine biosynthetic pathway of *Wolinella succinogenes* in a genetically modified microorganism producing methionine improves the methionine production and overcomes the problem of the cysteine production pathway optimization.

## SUMMARY OF THE INVENTION

The invention relates to a recombinant microorganism which produces methionine by fermentation wherein said microorganism expresses functional genes encoding a thiocarboxylated protein, a polypeptide having an homoserine O-acetyltransferase activity without feedback inhibition by methionine and/or S-adenosylmethionine and a polypeptide having O-acetylhomoserine sulfhydrylase activity. Method for the fermentative production of methionine comprising culturing said recombinant microorganism in an appropriate culture medium and recovering methionine from the culture medium is also an object of the invention.

## DESCRIPTION OF THE DRAWINGS

**Figure 1:** Comparative Methionine biosynthesis pathway in *Escherichia coli* and in *Wolinella succinogenes* with the protein-thiocarboxylate HcyS.

## DETAILED DESCRIPTION OF THE INVENTION

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting, which will be limited only by the appended claims.

Furthermore, the practice of the present invention employs, unless otherwise indicated, conventional microbiological and molecular biological techniques within the skill of the art. Such techniques are well known to the skilled worker, and are explained fully in the literature.

It must be noted that as used herein and in the appended claims, the singular forms "a", "an", and "the" include plural reference unless the context clearly dictates otherwise. Thus, for example, a reference to "a microorganism" includes a plurality of such microorganisms, and a reference to "an endogenous gene" is a reference to one or more endogenous genes, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

In the claims that follow and in the consecutive description of the invention, except where the context requires otherwise due to express language or necessary implication, the word "comprise", "contain", "involve" or "include" or variations such as "comprises", "comprising", "containing", "involved", "includes", "including" are used in an inclusive sense, i.e. to specify the presence of the stated features but not to preclude the presence or addition of further features in various embodiments of the invention.

The term "methionine" and "L-methionine" designate the essential sulfur-containing amino-acid with chemical formula $HO_2CCH(NH_2)CH_2CH_2SCH_3$ and CAS number 59-51-8 or 63-68-3 for the specific L-isomer.

The term "microorganism", as used herein, refers to a living microscopic organism, which may be a single cell, or a multicellular organism and which can generally be found in nature. In the context of the present invention, the microorganism is preferably a bacterium, yeast or fungus. More preferably, the microorganism of the invention is selected among *Enterobacteriaceae, Bacillaceae, Streptomycetaceae, Corynebacteriaceae* and yeast. Even more preferably, the microorganism of the invention is a species of *Escherichia, Klebsiella, Thermoanaerobacterium, Corynebacterium or Saccharomyces.* Yet, even more preferably, the microorganism of the invention is selected from *Escherichia coli, Klebsiella pneumoniae, Thermoanaerobacterium thermosaccharolyticum, Corynebacterium glutamicum* and *Saccharomyces cerevisiae.* Most preferably, the microorganism of the invention is either the species *Escherichia coli* or *Corynebacterium glutamicum.*

The term "recombinant microorganism", "genetically modified microorganism", or "genetically engineered microorganism", as used herein, refers to a microorganism as defined above that is not found in nature and therefore genetically differs from its natural counterpart. In other words, it refers to a microorganism that is modified by introduction and/or by deletion and/or by modification of its genetic elements. Such modification can be performed by genetic engineering, by forcing the development and evolution of new metabolic pathways by culturing the microorganism under specific selection pressure, or by combining both methods (see, e.g. WO2005/073364 or WO2008/116852).

A microorganism genetically modified for the production of methionine according to the invention therefore means that said microorganism is a recombinant microorganism as defined above that is capable of producing methionine. In other words, said microorganism has been genetically modified to allow higher productions of methionine than the non-modified microorganism.

According to the invention, the amount of methionine produced by the recombinant microorganism of the invention, and particularly the methionine yield (ratio of methionine produced per carbon source, in gram/gram or mol/mol), is higher in the modified microorganism compared to the corresponding unmodified microorganism.

In the context of the invention, "non-modified microorganism" and "unmodified microorganism" means a microorganism which does not contain any genetic modification of gene(s) involved in methionine production.

The modified microorganisms of the invention are optimized for methionine production and further genetically modified for expressing functional genes encoding a thiocarboxylated protein, a polypeptide having an homoserine O-acetyltransferase activity without feedback inhibition by methionine and/or S-adenosylmethionine and a polypeptide having O-acetylhomoserine sulfhydrylase activity. The terms "genetically modified microorganism producing methionine" or "methionine-producing microorganism" or "microorganism genetically modified for the production of methionine" or "microorganism optimized for the production of methionine" or "recombinant L-methionine producing strain" and expression derived thereof designate a microorganism as defined above producing higher levels of methionine than the non-modified microorganism. Microorganisms optimized for methionine production are well known in the art, and have been disclosed in particular in patent applications WO2005/111202, WO2007/077041, WO2009/043803, WO2010/020681, WO2011/073738, WO2011/080542, WO2011/080301, WO2012/055798, WO2013/001055, WO2013/190343, WO2015/028675 and WO2015/028674. For the sake of clarity the genetically modified microorganism producing methionine and expressing the thiocarboxylated protein, the homoserine O-acetyltransferase and the O-acetylhomoserine sulfhydrylase is named in this disclosure "recombinant microorganism of the invention" or "microorganism of the invention" and expression derived thereof.

As further explained below, the genetically modified microorganism producing methionine and the microorganism of the invention can be genetically modified by modulating the expression level of one or more endogenous genes, and/or by expressing one or more heterologous genes in said microorganism.

By "modulating", it is meant herein that the expression level of said gene is up-regulated, downregulated, or even completely abolished by comparison to its natural expression level. Such modulation can therefore result in an enhancement of the activity of the gene product, or alternatively, in a lower or null activity of the endogenous gene product.

By "gene", it is meant herein a nucleic acid molecule or polynucleotide that codes for a particular protein (i.e. polypeptide), or in certain cases, for a functional or structural RNA molecule. In the context of the present invention, the genes referred herein encode proteins, such as enzymes.

The term "functional gene" means that the expression of the gene is functional that is to say that the nucleotidic sequence contains all elements allowing gene transcription and gene translation and potentially excretion of the protein encoded by said gene.

The terms "encoding" or "coding" refer to the process by which a polynucleotide, (i.e. a gene), through the mechanisms of transcription and translation, produces an amino-acid sequence. Genes according to the invention are either endogenous genes or exogenous.

The term "endogenous gene" refers herein to a gene that is naturally present in the microorganism.

An endogenous gene can be overexpressed by introducing heterologous sequences which favour upregulation in addition to endogenous regulatory elements or by substituting those endogenous regulatory elements with such heterologous sequences, or by introducing one or more supplementary copies of the endogenous gene into the chromosome or a plasmid within the microorganism. Endogenous gene activity and/or expression level can also be modified by introducing mutations into their coding sequence to modify the gene product. A deletion of an endogenous gene can also be performed to inhibit totally its expression within the microorganism. Another way to modulate the expression of an endogenous gene is to exchange its promoter (i.e. wild type promoter) with a stronger or weaker promoter to up or down regulate the expression level of this gene. Promoters suitable for such purpose can be homologous or heterologous and are well-known in the art. It is within the skill of the person in the art to select appropriate promoters for modulating the expression of an endogenous gene.

In addition, or alternatively, a microorganism can be genetically modified to express one or more exogenous genes, provided that said genes are introduced into the microorganism with all the regulatory elements necessary for their expression in the host microorganism. The modification or "transformation" of microorganisms with exogenous DNA is a routine task for those skilled in the art.

By "exogenous gene" or "heterologous gene", it is meant herein that said gene is not naturally occurring in the microorganism. In order to express an exogenous gene in a microorganism, such gene can be directly integrated into the microorganism chromosome, or be expressed extra-chromosomally by plasmids or vectors within the microorganism. A variety of plasmids, which differ in respect of their origin of replication and of their copy number in a cell, are well known in the art and can be easily selected by the skilled practitioner for such purpose. Exogenous genes according to the invention are advantageously homologous genes.

In the context of the invention, the term "homologous gene" or "homolog" not only refers to a gene inherited by two species (i.e. microorganism species) by a theoretical common genetic ancestor, but also includes genes which may be genetically unrelated that have, nonetheless, evolved to encode proteins which perform similar functions and/or have similar structure (i.e. functional homolog). Therefore the term "functional homolog" refers herein to a gene that encodes a functionally homologous protein.

Using the information available in databases such as Uniprot (for proteins), Genbank (for genes), or NCBI (for proteins or genes), those skilled in the art can easily determine the sequence of a specific protein and/or gene of a microorganism, and identify based on this sequence the one of equivalent genes, or homologs, in another microorganism. This routine work can be performed by a sequence alignment of a specific gene sequence of a microorganism with gene sequences or the genome of other microorganisms, which can be found in the above mentioned databases. Such sequence alignment can advantageously be performed using the BLAST algorithm developed by Altschul *et al.* (1990). Once a sequence homology has been established between those sequences, a consensus sequence can be derived and used to design degenerate probes in order to clone the corresponding homolog gene of the related microorganism. These routine methods of molecular biology are well known to those skilled in the art.

It shall be further understood that, in the context of the present invention, should an exogenous gene encoding a protein of interest be expressed in a specific microorganism, a synthetic version of this gene is preferably constructed by replacing non-preferred codons or less preferred codons with preferred codons of said microorganism which encode the same amino acid. It is indeed well-known in the art that codon usage varies between microorganism species, which may impact the expression level of the protein of interest. To overcome this issue, codon optimization methods have been developed, and are extensively described by Graf *et al.* (2000), Deml *et al.* (2001) and Davis & Olsen (2011). Several software have notably been developed for codon optimization determination such as the GeneOptimizer® software (Lifetechnologies) or the OptimumGene™ software of GenScript. In other words, the exogenous gene encoding a protein of interest is preferably codon-optimized for expression in a specific microorganism.

The microorganism according to the invention can also be genetically modified to increase or decrease the expression of one or more genes.

The term "decrease the expression" or "attenuation of expression" according to the invention denotes the partial or complete suppression of the expression of the corresponding gene, which is then said to be 'decreased' or 'attenuated'. This suppression of expression can be either an inhibition of the expression of the gene, a deletion of all or part of the promoter region necessary for the gene expression, a deletion of all or part of the coding region of the gene, or the exchange of the wild type promoter by a weaker natural or synthetic promoter or by an inducible promoter. The man skilled in the art knows a variety of promoters which exhibit different strength and which promoter to use for a weak or an inducible genetic expression. Preferentially, the attenuation of a gene is essentially the complete deletion of that gene, which can be replaced by a selection marker gene that facilitates the identification, isolation and purification of the strains according to the invention. A gene is inactivated preferentially by the technique of homologous recombination (Datsenko & Wanner, 2000).

The terms "increased expression", "enhanced expression" or "overexpression" and grammatical equivalents thereof, are used interchangeably in the text and have a similar meaning. These terms mean that the expression of an endogenous or exogenous gene or the production of an enzyme or a protein is increased compared to the non modified microorganism leading to an increase in the intracellular concentration of a ribonucleic acid, a protein or an enzyme compared to the non modified microorganism. The man skilled in the art knows different means and methods to measure ribonucleic acid concentration or protein concentration in the cell including for instance use of Reverse Transcription Polymerase Chain Reaction (RT-PCR) to determine ribonucleic acid concentration and use of specific antibody to determine concentration of specific protein.

Increase production of a protein or an enzyme is obtained by increasing expression of the gene encoding said protein or enzyme by several techniques well known by the man skilled in the art.

In the context of the present invention, the terms "overexpress", "overexpression" or "overexpressing" could be used to designate an increase in transcription of a gene in a microorganism.

Increasing the transcription of a gene, whether endogenous or exogenous, can be achieved by increasing the number of its copies within the microorganism and/or by using a promoter leading to a higher level of expression of the gene compared to the wild type promoter.

As indicated above, to increase the number of copies of a gene in the microorganism, said gene can be encoded chromosomally or extra-chromosomally. When the gene of interest is to be encoded on the chromosome, several copies of the gene can be introduced on the chromosome by methods of genetic recombination, which are well-known to in the art (e.g. gene replacement). When the gene is to be encoded extra-chromosomally in the microorganism, it can be carried by different types of plasmid that differ in respect to their origin of replication depending on the microorganism in which they can replicate, and by their copy number in the cell. The microorganism transformed by said plasmid can contain 1 to 5 copies of the plasmid, or about 20 copies of it, or even up to 500 copies of it, depending on the nature of the plasmid. Examples of low copy number plasmids which can replicate in *E.coli* include, without limitation, the pSC101 plasmid (tight replication), the RK2 plasmid (tight replication), as well as the pACYC and pRSF1010 plasmids, while an example of high copy number plasmid which can replicate in *E. coli* is pSK bluescript II.

Promoters which can increase the expression level of a gene are also well-known to the skilled person in the art, and can be homologous (originating from same species) or heterologous (originating from a different species or artificial promoter). Examples of such promoters include, without limitation, the promoters P*trc,* P*tac,* P*lac,* and $P_R$ and $P_L$ of the lambda phage. These promoters can also be induced ("inducible promoters") by a particular compound or by specific external condition like temperature or light.

The terms "overproduce", "overproduction" or "overproducing" could also be used to designate an increase in the translation of a mRNA in a microorganism.

Increasing translation of the mRNA can be achieved by modifying the Ribosome Binding Site (RBS). A RBS is a sequence on mRNA that is bound by the ribosome when initiating protein translation. It can be either the 5' cap of a mRNA in eukaryotes, a region 6-7 nucleotides upstream of the start codon AUG in prokaryotes (called the Shine-Dalgarno sequence), or an internal ribosome entry site (IRES) in viruses. By modifying this sequence, it is possible to change the protein translation initiation rate, to proportionally alter its production rate, and control its level activity inside the cell. It is also possible to optimize the strength of a RBS sequence to achieve a targeted translation initiation rate by using the software RBS CALCULATOR (Salis, 2011). It is within the skill of the person in the art to select the RBS sequence based on the nature of the mRNA.

The term "activity" of an enzyme is used interchangeably with the term "function" and designates, in the context of the invention, the reaction that is catalyzed by the enzyme. The man skilled in the art knows how to measure the enzymatic activity of said enzyme.

The term "increased activity" or "enhanced activity" designates an enzymatic activity that is superior to the enzymatic activity of the non modified microorganism. Increasing such activity can be obtained by improving the protein catalytic efficiency, by decreasing protein turnover, by decreasing messenger RNA (mRNA) turnover in addition to the techniques described above for increasing transcription of the gene encoding protein or enzyme, or increasing translation of the mRNA.

Improving the protein catalytic efficiency means increasing the kcat and/or decreasing the Km for a given substrate and/or a given cofactor, and/or increasing the Ki for a given inhibitor. kcat, Km and Ki are Michaelis-Menten constants that the man skilled in the art is able to determine (Segel, 1993). Decreasing protein turnover means stabilizing the protein. Methods to improve protein catalytic efficiency and/or decrease protein turnover are well known from the man skilled in the art. Those include rational engineering with sequence and/or structural analysis and directed mutagenesis, as well as random mutagenesis and screening. Mutations can be introduced by site-directed mutagenesis by conventional methods such as Polymerase Chain Reaction (PCR), by random mutagenesis techniques, for example via mutagenic agents (Ultra-Violet rays or chemical agents like nitrosoguanidine (NTG) or ethylmethanesulfonate (EMS)) or DNA shuffling or error-prone PCR. Stabilizing the protein can also be achieved by adding a "tag" peptide sequence either at the N-terminus or the C-terminus of the protein. Such tags are well known in the art, and include, among others, the Glutathione-S-Transferase (GST).

Decreasing mRNA turnover can be achieved by modifying the gene sequence of the 5'-untranslated region (5'-UTR) and/or the coding region, and/or the 3'-UTR (Carrier and Keasling, 1999).

The terms "attenuated activity" or "reduced activity" of an enzyme mean either a reduced specific catalytic activity of the protein obtained by mutation in the aminoacids sequence and/or decreased concentrations of the protein in the cell obtained by mutation of the nucleotidic sequence or by deletion of the coding region of the gene as described above.

Decreasing the activity of a protein can mean either decreasing its specific catalytic activity and/or decreasing expression of the corresponding gene in the cell by way of mutation, suppression, insertion or modification of single or multiple residues in a polynucleotide leading to alterations arising within a protein-encoding region of a gene as well as alterations in regions outside of a protein-encoding sequence such as, but not limited to, regulatory or promoter sequences. The alteration may be a mutation of any type and for instance: a point mutation, a frame-shift mutation, a nonsense mutation, an insertion or a deletion of part or all of a gene as described above.

The terms "feedback sensitivity" or "feedback inhibition" refer to a cellular mechanism control in which one or several enzymes that catalyse the production of a particular substance in the cell are inhibited or less active when that substance has accumulated to a certain level. So the terms "reduced feedback sensitivity" or "reduced feedback inhibition" or "without feedback inhibition" mean that the activity of such a mechanism is decreased or suppressed compared to a non modified microorganism. The man skilled in the art knows how to modify the enzyme to obtain this result. Such modifications have been described in the patent application WO 2005/111202 or in the patent US 7,611,873.

The present invention is directed to a genetically modified microorganism producing methionine by fermentation, wherein said microorganism is further genetically modified for

- overexpressing the heterologous functional genes coding for the protein thiocarboxylate dependent methionine biosynthetic pathway of *Wolinella succinogenes, hcyS, hcyD, hcyF* and *sir* from *Wolinella succinogenes* [PCT text, p. 11, lines 32-33],
  and for expressing functional genes encoding
- a polypeptide having an homoserine O-acetyltransferase activity without feedback inhibition by methionine and/or S-adenosylmethionine and,
- a polypeptide having O-acetylhomoserine sultbydrylase activity.

In a first aspect of the invention the recombinant microorganism of the invention expresses functional genes encoding a thiocarboxylated protein as sulfur donor in the methionine biosynthetic pathway.

Thiocarboxylated proteins or protein thiocarboxylates are proteins wherein the carboxylic acid function at C-terminal position has one or both of the oxygen replaced by sulfur (R-COSH, R-CSOH, R-CSSH). These proteins are important intermediates in a variety of biochemical sulfide transfer reactions. These proteins are members of a growing family of biosynthetic sulfide donors and are involved in a variety of biosynthetic pathways.

In the methionine biosynthetic pathway the thiocarboxylated protein reacts with acetylhomoserine to form the complex thiocarboxylated protein-homocysteine by the action of O-acetylhomoserine sultbydrylase. Then the complex is hydrolysed to liberate homocysteine finally methylated to form methionine as described in figure 1.

The recombinant microorganism of the invention overexpresses the genes *hcyS, hcyD, hcyF* and *sir* from *Wolinella succinogenes* and encoding the thiocarboxylated protein HcyS. The *hcyS* gene as set forth in SEQ ID NO : 1 encodes the protein HcyS-Ala as set forth in SEQ ID NO : 2. The *hcyD* gene as set forth in SEQ ID NO : 3 encodes a metalloprotease as set forth in SEQ ID NO : 4 involved in C-terminal processing of HcyS-Ala by removing the C-terminal alanine from HcyS-Ala for giving HcyS protein but also for an enzyme catalyzing the release of homocysteine from HcyS homocysteine. The *hcyF* gene as set forth in SEQ ID NO : 5 encodes an enzyme as set forth in SEQ ID NO : 6 catalyzing the adenylation of HcyS protein. HcyS acyl-adenylate then undergoes nucleophilic substitution by bisulfide produced by the protein Sir as set forth SEQ ID NO : 7 encoded by the *sir* gene as set forth in SEQ ID NO : 8.

In a second aspect of the invention the recombinant microorganism of the invention expresses functional genes encoding for a polypeptide having an homoserine O-acetyltransferase activity without feedback inhibition by methionine and/or S-adenosylmethionine. This enzyme activity allows the cell to accumulate O-acetylhomoserine able to react with the thiocarboxylated protein described above.

A polypeptide having an homoserine O-acetyltransferase activity is a polypeptide having an enzyme activity catalyzing the chemical reaction:

$$\text{acetyl-CoA + L-homoserine} \rightleftarrows \text{CoA + O-acetyl-L-homoserine}$$

Thus the two substrates of this enzyme are acetyl-CoA and L-homoserine, whereas its two products are CoA and O-acetyl-L-homoserine.

This enzyme belongs to the family of transferases (EC 2 enzyme), specifically those acyltransferases transferring groups other than aminoacyl groups (EC 2.3 enzyme). The systematic name of this enzyme class is acetyl-CoA:L-homoserine O-acetyltransferase. Other names in common use include homoserine acetyltransferase, homoserine transacetylase, homoserine-O-transacetylase, and L-homoserine O-acetyltransferase or more currently MetX protein. This enzyme participates in methionine metabolism and sulfur metabolism.

The homoserine O-acetyltransferase enzyme activity may be controlled by a feedback inhibition mechanism with methionine and/or S-adenosylmethionine or not that is to say that feedback inhibition by methionine and/or S-adenosylmethionine is reduced or suppressed.

The enzyme having homoserine O-acetyltransferase activity to be present in the recombinant microorganism of the invention has no feedback inhibition by methionine and/or S-adenosylmethionine, the activity of said enzyme being not inhibited by methionine and/or S-adenosymethionine: the O-acetylhomoserine formation pool is not suppressed or decreased by a methionine and/or S-adenosylmethionine concentration level.

In another embodiment of the invention the gene *metX* encoding the enzyme having homoserine O-acetyltransferase activity is endogenous or heterologous.

Preferably, the gene encoding the enzyme having homoserine O-acetyltransferase activity is heterologous and may originate from a variety of microorganisms. Microorganisms from which a gene *metX* encoding an enzyme having homoserine O-acetyltransferase activity can be obtained include *Corynebacterium species, Leptospira species, Deinococcus species, Pseudomonas species* or *Mycobacterium species* but are not limited thereto. Preferably the enzyme having homoserine O-acetyltransferase activity may be encoded by a gene *metX* originating from a strain selected from a group consisting of *Corynebacterium glutamicum, Leptospira meyerei, Deinococcus radiodurans, Pseudomonas aeruginosa* and *Mycobacterium smegmatis.* The *metX* gene as set forth in SEQ ID NO : 9 originating from *Leptospira meyeri* encodes an enzyme having an homoserine O-acetyltransferase activity without feedback inhibition by methionine and/or S-adenosylmethionine as set forth in SEQ ID NO : 10 (Bourhy *et al,* 1997). Other homoserine O-acetyltransferases showing resistance to feedback inhibition can be obtained by techniques well known by the man skilled in the art and are notably described in WO2005111202, US8551742, EP2290051 and WO2008013432 patent applications.

Most preferably the recombinant microorganism of the invention expresses the gene *metX* from *Leptospira meyeri* encoding an enzyme having an homoserine O-acetyltransferase activity without feedback inhibition by methionine and/or S-adenosylmethionine and even most preferably said gene is overexpressed.

In a third aspect of the invention the recombinant microorganism of the invention expresses functional genes encoding a polypeptide having O-acetylhomoserine sulfhydrylase activity.

This enzyme catalyzes the reaction of thiocarboxylated protein HcyS with O-acetylhomoserine to form the complex HcyS-Homocysteine and allows incorporation of the sulfur group from thiocarboxylated protein HcyS to acetylhomoserine.

This enzyme belongs to the family of transferases (EC 2 enzyme) that transfer specific functional group (e.g. a methyl or glycosyl group) from one molecule (called the donor) to another (called the acceptor). Specifically the enzyme transfers alkyl or aryl groups, other than methyl groups (EC 2.5.1). The specific name of this enzyme is *O*-acetyl-L-homoserine:methanethiol 3-amino-3-carboxypropyltransferase. Other names in common use include *O*-acetyl-L-homoserine acetate-lyase (adding methanethiol), *O*-acetyl-L-homoserine sulfhydrolase, *O*-acetylhomoserine (thiol)-lyase, *O*-acetylhomoserine sulfhydrolase and methionine synthase or more currently MetY protein. This enzyme participates in methionine metabolism and sulfur metabolism.

In one embodiment the gene *metY* encoding the enzyme having O-acetylhomoserine sulfhydrylase activity is endogenous or heterologous.

Preferably, the gene encoding the enzyme having O-acetylhomoserine sulfhydrylase activity is heterologous.

Most preferably the microorganism of the invention expresses the gene *metY* from *Wolinella succinogenes* as set forth in SEQ ID NO : 11 encoding a polypeptide having O-acetylhomoserine sulfhydrylase activity as set forth in SEQ ID NO : 12, and even most preferably said gene is overexpressed. Said overexpression may also be optimized by expressing a modified *metY* gene from *Wolinella succinogenes* as set forth in SEQ ID NO : 13, thus encoding a polypeptide having O-acetylhomoserine sulfhydrylase activity as set forth in SEQ ID NO : 14.

Preferably, in the recombinant microorganism of the invention, the gene encoding a polypeptide having homoserine O-acetyltransferase activity and the gene encoding a polypeptide having O-acetylhomoserine sulfhydrylase activity are heterologous.

Preferably the microorganism used in the invention is able to produce the L-methionine amino acid. More preferably the genetically modified microorganism producing methionine of the invention is optimized for the production of L-methionine.

Genes involved in methionine production in a microorganism are well known in the art, and comprise genes involved in the methionine specific biosynthesis pathway as well as genes involved in precursor-providing pathways and genes involved in methionine consuming pathways.

Efficient production of methionine requires the optimisation of the methionine specific pathway and several precursor-providing pathways. L-Methionine producing strains have been described in patent applications WO2005/111202, WO2007/077041 and WO2009/043803, WO2010/020681, WO2011/073738, WO2011/080542, WO2011/080301, WO2012/055798, WO2013/001055, WO2013/190343, WO2015/028675 and WO2015/028674.

For improving the production of L-methionine, the microorganism genetically modified for the production of methionine may exhibit:

- an increased expression of at least one gene selected in the group consisting of:

    - *cysP* which encodes a periplasmic sulfate binding protein, as described in WO2007/077041 and in WO2009/043803,
    - *cysU* which encodes a component of sulfate ABC transporter, as described in WO2007/077041 and in WO2009/043803,

- *cysW* which encodes a membrane bound sulfate transport protein, as described in WO2007/077041 and in WO2009/043803,
- *cysA* which encodes a sulfate permease, as described in WO2007/077041 and in WO2009/043803,
- *cysM* which encodes an O-acetyl serine sulfhydralase, as described in WO2007/077041 and in WO2009/043803,
- *cysI* and *cysJ* encoded respectively the alpha and beta subunits of a sulfite reductase as described in WO2007/077041 and in WO2009/043803. Preferably *cysI* and *cysJ* are overexpressed together,
- *cysH* which encodes an adenylylsulfate reductase, as described in WO2007/077041 and in WO2009/043803.

Increasing C1 metabolism is also a modification that leads to improved methionine production. It relates to the increase of the activity of at least one enzyme involved in the C1 metabolism chosen among GcvTHP, Lpd, MetF or MetH. In a preferred embodiment of the invention, the one carbon metabolism is increased by enhancing the expression and/or the activity of at least one of the following:

- *gcvT, gcvH, gcvP,* and *lpd,* coding for the glycine cleavage complex, as described in patent application WO 2007/077041. The glycine-cleavage complex (GCV) is a multienzyme complex that catalyzes the oxidation of glycine, yielding carbon dioxide, ammonia, methylene-THF and a reduced pyridine nucleotide. The GCV complex consists of four protein components, the glycine dehydrogenase said P-protein (GcvP), the lipoyl-GcvH-protein said H-protein (GcvH), the aminomethyltransferase said T-protein (GcvT), and the dihydrolipoamide dehydrogenase said L-protein (GcvL or Lpd). P-protein catalyzes the pyridoxal phosphate-dependent liberation of CO2 from glycine, leaving a methylamine moiety. The methylamine moiety is transferred to the lipoic acid group of the H-protein, which is bound to the P-protein prior to decarboxylation of glycine. The T-protein catalyzes the release of NH3 from the methylamine group and transfers the remaining C1 unit to THF, forming methylene-THF. The L protein then oxidizes the lipoic acid component of the H-protein and transfers the electrons to $NAD^+$, forming NADH;
- *MetF* encoding a methylenetetrahydrofolate reductase, as described in patent application WO2007/07704. In a specific embodiment of the invention, the activity of MetF is enhanced by overexpressing the gene *metF* and/or by optimizing the translation. In another specific embodiment of the invention, overexpression of *metF gene* is achieved by expressing the gene under the control of a strong promoter belonging to the *Ptrc* family promoters, or under the control of an inducible promoter, like a temperature inducible promoter $P_R$ as described in application WO2011/073738. According to another embodiment of the invention, optimisation of the translation of the protein MetF is achieved by using a RNA stabiliser. Other means for the overexpression of a gene are known to the expert in the field and may be used for the overexpression of the *metF* gene.

The overexpression of at least one of the following genes involved in serine biosynthesis also reduces the production of by-product isoleucine:

- *serA* which encodes a phosphoglycerate dehydrogenase, as described in WO2007/077041 and in WO2009/043803,
- *serB* which encodes a phosphoserine phosphatase, as described in WO2007/077041 and in WO2009/043803,
- *serC* which encodes a phosphoserine aminotransferase, as described in WO2007/077041 and in WO2009/043803.

The overexpression of the following genes has already been shown to improve the production of methionine:

- *thrA* or *thrA* alleles which encode aspartokinases/homoserine dehydrogenase with reduced feed-back inhibition to threonine (*thrA**), as described in WO2009/043803 and WO2005/111202,
- *metL* encoding for a polypeptide having bifunctionnal aspartokinase/homoserine dehydrogenase.
- *ptsG* which encodes the glucose-specific phosphoenolpyruvate (PEP) phosphotransferase system (PTS) permease, as described in WO 2013/001055,
- *metH* which encodes a cobalamin-dependent methionine synthase, as described in WO2015/028674,
- *metE* which encodes a cobalamin-independent methionine synthase, as described in WO2013/190343,
- *fldA* which encodes an essential flavodoxin containing FMN as a prosthetic group which interacts with Fpr and MetH proteins, as described in WO2015/028674,
- *fpr* which encodes a flavodoxin NADP+ reductase required for the activation of the methionine synthase MetH as described in WO2015/028674. The reductase uses non covalently bound FAD as a cofactor,
- *pntAB* operon which encodes respectively the $\alpha$-subunit and the an inner membrane protein with nine predicted transmembrane domains of the membrane bound proton translocating pyridine nucleotide transhydrogenase, as described in WO 2012/055798,

- *ygaZH* operon which encodes a member of the branched chain amino acid exporter (LIV-E) family responsible for export of L-valine and L-methionine,
- *pyc* which encodes pyruvate carboxylase as described in patent application WO 2012/055798. Increasing activity of pyruvate carboxylase is obtained by overexpressing the corresponding gene or modifying the nucleic sequence of this gene to express an enzyme with improved activity. In another embodiment of the invention, the *pyc* gene is introduced on the chromosome in one or several copies by recombination or carried by a plasmid present at least at one copy in the modified microorganism. The *pyc* gene originates from *Rhizobium etli, Bacillus subtilis, Pseudomonas fluorescens, Lactococcus lactis* or *Corynebacterium* species. In a preferred embodiment, the microorganism of the invention overexpresses *pyc* gene from *Rhizobium etli.*

- and/or an decrease of the expression of at least one of the following genes:

  - *pykA* which encodes a pyruvate kinase, as described in WO2007/077041 and in WO2009/043803,
  - *pykF* which encodes a pyruvate kinase, as described in WO2007/077041 and in WO2009/043803,
  - *purU* which encodes a formyltetrahydrofolate deformylase, as described in WO2007/077041 and in WO2009/043803,
  - *yncA* which encodes a N-acetyltransferase, as described in WO 2010/020681,
  - *metJ* which encodes a repressor of the methionine biosynthesis pathway, as described in WO2005/111202. The repressor protein MetJ is responsible for the down-regulation of the methionine regulon as was suggested in patent application JP2000/157267,
  - *udhA* which encodes a soluble pyridine nucleotide transhydrogenase which catalyses essentially the oxidation of NADPH into $NADP^+$ via the reduction of $NAD^+$ into NADH as described in patent application WO 2012/055798.
  - *dgsA* which encodes a transcriptional dual regulator that controls the expression of a number of genes encoding enzymes of the phosphotransferase (PTS) and phosphoenolpyruvate (PEP) systems, as described in WO 2013/001055,
  - *sgrS* which encodes a small RNA which regulates post-transcriptionally the abundance of PtsG, as described in WO 2013/001055
  - *sgrT* which encodes a regulator which plays a role in the glucose-phosphate stress response, regulating the activity of PtsG, as described in WO 2013/001055
  - *metNIQ* operon which encodes for the subunits of the ABC transporter involved in the uptake of methionine.

Genes may be expressed under control of an inducible promoter. Patent application WO2011/073738 describes a L-methionine producing strain that expresses a *thrA* allele with reduced feed-back inhibition to threonine under the control of an inducible promoter (*thrA**). In a specific embodiment of the invention, the *thrA* or *thrA* allele, *pyc, pntAB, ygaZH or ptsG* genes are under control of a temperature inducible promoter. In a most preferred embodiment, the temperature inducible promoter used belongs to the family of $P_R$ or $P_L$ promoters.

In a particular embodiment of the invention, the overexpressed genes are at their native position on the chromosome or are integrated at a non-native position. For an optimal L-methionine production, several copies of the gene may be required, and these multiple copies are integrated into specific loci, whose modification does not have a negative impact on methionine production.

Examples for locus into which a gene may be integrated, without disturbing the metabolism of the cell, are disclosed in patent applications WO2011/073122, WO2011/073738 and WO2012/055798.

In one preferred embodiment of the invention the genetically modified microorganism producing methionine overexpresses at least one of the following genes: *thrA* or *thrA* allele encoding a polypeptide having aspartokinase/homoserine dehydrogenase activity with reduced feedback inhibition to threonine (*thrA**), *metL* encoding a polypeptide having bifunctionnal aspartokinase/homoserine dehydrogenase, *metE* encoding a polypeptide having cobalamin-independent methionine synthase or *metH* encoding a polypeptide having cobalamin-dependent methionine synthase. More preferably the genetically modified microorganism producing methionine overexpresses the genes *metH* and *metL.* Even more preferably the genetically modified microorganism producing methionine overexpresses the genes *thrA*, metH and metL.*

In another preferred embodiment of the invention the *metJ* gene is deleted in order to avoid repression of methionine regulon. In this case neither the endogenous genes *metL, metB, metE* and/or *metH,* nor exogenous genes under the control of endogenous promoter belonging to the methionine regulon are repressed by MetJ protein.

In another preferred embodiment, the *metJ* gene is expressed but in this case the promoters of endogenous genes belonging to the MetJ regulon and the promoters used to control exogenous gene expression and which belong to the MetJ regulon are exchanged. Promoters suitable for such purpose can be homologous or heterologous and are well-known in the art.

In a particular aspect, the recombinant microorganism of the invention comprises the following genetic modifications:

- the expression of the genes *hcyS, hcyD, hcyF* and *sir* from *Wolinella succinogenes, metX* from *Leptospira meyeri* and *metY* from *Wolinella succinogenes* are enhanced

In addition to these modifications, the recombinant microorganism of the invention preferably further comprises:

◦ Increased expression of at least one the following genes: *pyc, ptsG, pntAB, cysP, cysU, cysW, cysA, cysM, cysJ, cysI, cysH, gcvT, gcvH, gcvP, lpd, glyA,* serA, *serB, serC, metF, fldA, fpr, metN, metI, metQ,* and/or
◦ Attenuated expression of at least one of the following genes: *metJ, pykA, pykF, purU, yncA, metE, dgsA, sgrS, sgrT, ygaZH* or *udhA.*

More particularly, the recombinant microorganism of the invention comprises the following genetic modifications:

- the expression of the genes *hcyS, hcyD, hcyF* and *sir* from *Wolinella succinogenes, metX* from *Leptospira meyeri* and *metY* from *Wolinella succinogenes* are enhanced
- the expression of the genes *cysPUWAM, cysJIH, thrA** and *metH* are enhanced.

Even more particularly, the recombinant microorganism of the invention comprises the following genetic modifications:

- the expression of the genes *hcyS, hcyD, hcyF* and *sir* from *Wolinella succinogenes, metX* from *Leptospira meyeri* and metYfrom *Wolinella succinogenes* are enhanced

- the expression of the genes *cysPUWAM, cysJIH, gcvTHP, thrA*, metF* and *metH* are enhanced, and,

- the expression of the genes *metJ* is attenuated.

Or:

- the expression of the genes *hcyS, hcyD, hcyF* and *sir* from *Wolinella succinogenes, metX* from *Leptospira meyeri* and *metY* from *Wolinella succinogenes* are enhanced

- the expression of the genes *cysPUWAM, cysJIH, gcvTHP, thrA* *, *metF* and *metH* are enhanced, and,

- the expression of the genes *metJ, pykA, pykF* and *purU* are attenuated .

Or:

- the expression of the genes *hcyS, hcyD, hcyF* and *sir* from *Wolinella succinogenes, metX* from *Leptospira meyeri* and *metY* from *Wolinella succinogenes* are enhanced

- the expression of the genes *cysPUWAM, cysJIH, gcvTHP, thrA**, *metF* and *metH* are enhanced, and,

- the expression of the genes *pykA, pykF* and *purU* are attenuated .

The microorganism of the invention preferably belongs to the family of *Enterobacteriaceae* or *Corynebacteriaceae,* and most preferably is *Escherichia coli.*

Finally, the present invention is related to a method for the fermentative production of methionine comprising culturing a genetically modified microorganism producing methionine as described above overexpressing the heterologous functional genes coding for the protein thiocarboxylate dependent methionine biosynthetic pathway of *Wolinella succinogenes, hcyS, hcyD, hcyF* and *sir* from *Wolinella succinogenes* and expressing a polypeptide having an homoserine O-acetyltransferease activity without feedback inhibition by methionine and/or S-adenosylmethionine and a polypeptide having O-acetylhomoserine sulfbydrylase activity and recovering methionine from said culture medium.

According to a specific aspect of the invention, the method is performed with a recombinant microorganism overexpressing *hcyS, hcyD, hcyF* and *sir* genes from *Wolinella succinogenes, metX* gene from *Leptospira meyeri* and *metY* gene from *Wolinella succinogenes.*

According to another specific aspect of the method, the microorganism further overexpresses at least one of the following genes: *thrA* or *thrA* allele encoding a polypeptide having aspartokinase/homoserine dehydrogenase activity with reduced feedback inhibition to threonine (*thrA**), *metL* encoding a polypeptide having bifunctionnal aspartokinase/homoserine dehydrogenase, *metE* encoding a polypeptide having cobalamin-independent methionine synthase or *metH* encoding a polypeptide having cobalamin-dependent methionine synthase.

According to the invention, the terms "fermentative process", "culture" or "fermentation" are used interchangeably to denote the growth of a given microorganism on an appropriate culture medium containing a carbon source, a source of sulfur and a source of nitrogen. The growth is generally performed in fermenters with an appropriate growth medium adapted to the microorganism being used.

In the fermentative process of the invention, the source of carbon is used simultaneously for:

- biomass production: growth of the microorganism by converting inter alia the carbon source of the medium, and,
- methionine production: transformation of the same carbon source into methionine by the biomass.

The two steps are concomitant, and the transformation of the source of carbon by the microorganism to grow results in the L-methionine production in the medium, since the microorganism comprises a metabolic pathway allowing such conversion.

An "appropriate culture medium" means herein a medium (e.g., a sterile, liquid media) comprising nutrients essential or beneficial to the maintenance and/or growth of the microorganism such as carbon sources or carbon substrates; nitrogen sources, for example peptone, yeast extracts, meat extracts, malt extracts, urea, ammonium sulfate, ammonium chloride, ammonium nitrate and ammonium phosphate; phosphorus sources, for example monopotassium phosphate or dipotassium phosphate; trace elements (e.g., metal salts) for example magnesium salts, cobalt salts and/or manganese salts; as well as growth factors such as amino acids and vitamins.

The term "source of carbon" according to the invention denotes any source of carbon that can be used by those skilled in the art to support the normal growth of a microorganism, which can be hexoses such as glucose, galactose or lactose; pentoses; monosaccharides; disaccharides such as sucrose (molasses), cellobiose or maltose; oligosaccharides such as starch or its derivatives; hemicelluloses; glycerol and combinations thereof. An especially preferred carbon source is glucose. Another preferred carbon source is sucrose.

In a particular embodiment of the invention, the carbon source is derived from renewable feed-stock. Renewable feed-stock is defined as raw material required for certain industrial processes that can be regenerated within a brief delay and in sufficient amount to permit its transformation into the desired product. Vegetal biomass treated or not, is an interesting renewable carbon source.

The source of carbon is fermentable, i.e. it can be used for growth by microorganisms.

The term "source of sulfur" according to the invention refers to sulfate, thiosulfate, hydrogen sulfide, dithionate, dithionite, sulfite, methylmercaptan, dimethylsulfide, dimethyl disulfide and other methyl capped sulfides or a combination of the different sources. Preferred sulfur source in the culture medium is sulfate or thiosulfate or a mixture thereof. Another preferred sulfur source is dimethyl disulfide.

The term "source of nitrogen" corresponds to either an ammonium salt or ammoniac gas. Nitrogen comes from an inorganic (e.g., $(NH_4)_2SO_4$) or organic (e.g., urea or glutamate) source. In the invention sources of nitrogen in culture are $(NH_4)_2HPO_4$, $(NH_4)_2S_2O_3$ and $NH_4OH$.

The culture may be performed in such conditions that the microorganism is limited or starved for an inorganic substrate, in particular phosphate and/or potassium. Subjecting an organism to a limitation of an inorganic substrate defines a condition under which growth of the microorganisms is governed by the quantity of an inorganic chemical supplied that still permits weak growth. Starving a microorganism for an inorganic substrate defines the condition under which growth of the microorganism stops completely due, to the absence of the inorganic substrate.

Those skilled in the art are able to define the culture conditions and the composition of culture medium for the microorganisms according to the invention. In particular the bacteria are fermented at a temperature between 20°C and 55°C, preferentially between 25°C and 40°C, and more specifically about 30°C for *C. glutamicum* and about 37°C for *E. coli.*

As an example of known culture medium for *E. coli,* the culture medium can be of identical or similar composition to an M9 medium (Anderson, 1946, Proc. Natl. Acad. Sci. USA 32:120-128), an M63 medium (Miller, 1992; A Short Course in Bacterial Genetics: A Laboratory Manual and Handbook for Escherichia coli and Related Bacteria, Cold Spring Harbor Laboratory Press, Cold Spring Harbor, New York) or a medium such as defined by Schaefer et al. (1999, Anal. Biochem. 270: 88-96).

As an example of known culture medium for *C. glutamicum,* the culture medium can be of identical or similar composition to BMCG medium (Liebl et al., 1989, Appl. Microbiol. Biotechnol. 32: 205-210) or to a medium such as described by Riedel et al. (2001, J. Mol. Microbiol. Biotechnol. 3: 573-583).

The method of the invention can be performed either in a batch process, in a fed-batch process or in a continuous process, and under aerobic, micro-aerobic or anaerobic conditions.

A fermentation "under aerobic conditions" means that oxygen is provided to the culture by dissolving gas into the liquid phase of the culture. This can be achieved by (1) sparging oxygen containing gas (e.g. air) into the liquid phase, or (2) shaking the vessel containing the culture medium in order to transfer the oxygen contained in the head space into the liquid phase. The main advantage of the fermentation under aerobic conditions is that the presence of oxygen as an electron acceptor improves the capacity of the strain to produce more energy under the form of ATP for cellular processes, thereby improving the general metabolism of the strain.

Micro-aerobic conditions can be used herein and are defined as culture conditions wherein low percentages of oxygen (e.g. using a mixture of gas containing between 0.1 and 10% of oxygen, completed to 100% with nitrogen) are dissolved into the liquid phase.

By contrast, "anaerobic conditions" are defined as culture conditions wherein no oxygen is provided to the culture medium. Strictly anaerobic conditions can be obtained by sparging an inert gas like nitrogen into the culture medium to remove traces of other gas. Nitrate can be used as an electron acceptor to improve ATP production by the strain and improve its metabolism.

In the invention, the fermentation is done in fed-batch mode. This refers to a type of fermentation in which supplementary growth medium is added during the fermentation, but no culture is removed until the end of the batch (except small volumes for samplings and HPLC/GCMS analysis). The process comprises two main steps; the first one which is a series of pre cultures in appropriate batch mineral medium and fed-batch mineral medium. Subsequently, a fermentor filled with appropriate minimal batch medium is used to run the culture with different fed-batch medium according to the desire production.

The method of the invention further comprises a step of recovering the methionine from the culture medium.

The action of "recovering methionine from the culture medium" designates the action of recovering methionine from the fermentation medium whatever its purity degree. "Recovering" means recovering the first product directly obtained from the fermentative process (fermentation must) which contains the product of interest (in this case methionine) and other co-products of the fermentation so with a more or less acceptable purity degree.

The "purifying" step consists of specifically purify the product of interest (in this case methionine) in order to obtain methionine with an improved purity degree.

Methionine might be recovered and purified by techniques and means well known by the man skilled in the art like distillation, ion-exchange chromatographic methods, precipitation, crystallisation or complexation with salts and particularly with calcium salts or ammonium salts.

The methods for the recovery and purification of the produced compounds are well known to those skilled in the art (see in particular WO 2005/007862, WO 2005/059155). Preferably, the step of recovering methionine comprises a step of concentration of methionine and/or its derivatives in the fermentation broth.

The amount of product in the fermentation medium can be determined using a number of methods known in the art, for example, high performance liquid chromatography (HPLC) or gas chromatography (GC). For example the quantity of methionine obtained in the medium is measured by HPLC after OPA/Fmoc derivatization using L-methionine (Sigma, Ref 64319) as a standard.

## EXAMPLES

The following experiments demonstrate how overexpression of genes encoding for proteins involved in production of the thiocarboxylated protein HcyS together with the overexpression of genes encoding for enzymes involved in production of acetyl-homoserine and HcyS-homocysteine in microorganisms such as *E. coli* improved methionine production.

In the examples given below, methods well known in the art were used to construct *E. coli* strains containing replicating vectors and/or various chromosomal insertions, deletions, and substitutions using homologous recombination well described by Datsenko & Wanner, (2000) for *E.coli.*

In the same manner, the use of plasmids or vectors to express or overexpress one or several genes in a recombinant microorganisms are well known by the man skilled in the art.

Examples of suitable *E.coli* expression vectors include pTrc, pACYC184, pBR322, pUC18, pUC19, pKC30, pRep4, pHS1, pHS2, pPLc236 etc...

## PROTOCOLS

Several protocols have been used to construct methionine producing strains described in the following examples.

Protocol 1 (Chromosomal modifications by homologous recombination, selection of recombinants and antibiotic cassette excision) and protocol 2 (Transduction of phage P1) used in this invention have been fully described in patent application WO2013/001055.

**Protocol 3:** Construction of recombinant plasmids

Recombinant DNA technology is well described and known by the man skilled in the art. Briefly, the DNA fragments are PCR amplified using oligonucleotides that the person skilled in the art is able to design and genomic DNA of the strain of interest is used as matrix. The DNA fragments and selected plasmid are digested with compatible restriction enzymes, ligated and then transformed in competent cells. Transformants are analysed and recombinant plasmids of interest are verified by DNA sequencing.

**EXAMPLE 1: Overproduction of the protein thiocarboxylate-dependent methionine biosynthesis pathway of *Wolinella succinogenes* in a recombinant L-methionine producing *E. coli* strain - Strain 1, and construction of strains 2 and 3.**

Methionine producing strain used in this application: Strain 1

Strain 1: The L-methionine producing strain used as recipient for the overproduction of protein thiocarboxylate-dependent methionine biosynthesis pathway of *Wolinella succinogenes* is MG1655 *metA**11 *DmetJ* P*trc*36-ARNmst27-*metF* P*trc-metH* P*trc*F-*cysJIH* P*trc*F-*cysPUWAM*P*trc*09-*gcvTHP DpykA* D*pykF* D*purU,* described in the previous patent application WO2009/043803, and named strain 1 in this present patent application This strain is a L-methionine producing *E. coli* strain which doesn't possess fully optimization of its endogenous cysteine production pathway. This strain is used as reference for comparison with the recombinant strain of the invention which possesses protein thiocarboxylate-dependent methionine biosynthesis pathway of *Wolinella succinogenes* and which is described in this present application.

Overproduction of the protein thiocarboxylate-dependent methionine biosynthesis pathway of *Wolinella succinogenes:* overexpression of *hcys, hcyF, hcyD* and *sir* from *Wolinella succinogenes*

The gene *hcyS* as set forth in SEQ ID NO : 1 encoding the sulfur transfer protein as set forth in SEQ ID NO : 2), the gene *hcyD* as set forth in SEQ ID NO : 3 encoding the metalloprotease as set forth in SEQ ID NO : 4, the gene *hcyF* as set forth in SEQ ID NO : 5 encoding the enzyme as set forth in SEQ ID NO : 6 that catalyses the adenylation of HcyS, , and the gene *sir* as set forth in SEQ ID NO : 8 encoding the sulfite reductase as set forth in SEQ ID NO 7, all from *Wolinella succinogenes* (ATCC29543D-5), were overexpressed in genetic background of strain 1.

The operon *hcySFD-sir* was overexpressed by using the same promoter as described for *cysE* gene into the pME101-*thrA*1-cysE* plasmid described in patent application WO2007/0770441, the ribosome binding site of each *hcySFD-sir* genes and the moderate plasmid copy number pCL1920 (Lerner & Inouye, 1990). More precisely, the *hcySFD-sir* operon, operatively linked to the chosen promoter, was cloned downstream of *thrA**1 gene into the pME101-*thrA**1 recombinant plasmid described in patent application WO2007/0770441. This plasmid was named pME1308.

Modification of the recombinant L-methionine producing *E. coli* strain with the alternative methionine biosynthesis pathway from *Wolinella succinogenes:* deletion of *metA**11 allele and overexpression of *metX and metY* genes from *Leptospira meveri* and *Wolinella succinogenes,* respectively

To produce acetyl-homoserine instead of succinyl-homoserine with strain 1, the *E. coli metA**11 gene coding for the homoserine O-succinyltransferase was deleted, and replaced by the overexpression of *metX* gene as set forth in SEQ ID NO : 9 from *Leptospira meyeri* (*Leptospira meyeri serovar semaranga* - DSM21536) encoding the homoserine O-acetyltransferase as set forth in SEQ ID NO : 10. Then, HcyS-homocysteine, which is the addition of acetyl-homoserine to HcyS thiocarboxylate (HcyS-COSH), is obtained by overexpressing the *Wolinella succinogenes metY* gene coding for the O-acetylhomoserine-sulfhydrylase.

Deletion of *metA**11 allele of *E. coli* strain 1 - Construction of strain 2

To delete *metA*11* allele from strain 1, the homologous recombination strategy described by Datsenko & Wanner, 2000 (according to Protocol 1) was used. A fragment carrying a resistance marker, flanked by DNA sequence homologous to the sequences up- and downstream of *metA*11* locus as set forth in SEQ ID NO : 15 and in SEQ ID NO : 16), was PCR amplified. The PCR product obtained was then introduced by electroporation into strain 1, in which the pKD46 vector had beforehand been introduced. The antibiotic resistant transformants were then selected and the deletion of the *metA**11 gene associated to the resistance cassette was verified by a PCR analysis with appropriate oligonucleotides. The strain retained was designated strain 2.

Overexpression of *metX* of *Leptospira meyeri*

The *metX* gene of *Leptospira meyeri* was overexpressed by using the same promoter and ribosome binding site as described for *metA*11* gene into the *pCL1920-TTadc-CI857-PlambdaR*(-35)-thrA*1-cysE-PgapA-metA*11* plasmid described in the patent application WO2011/073122 (Example 1) and the moderate plasmid copy number pCL1920 (Lerner & Inouye, 1990). More precisely, the *metX* gene, operatively linked to the chosen promoter, was cloned downstream of the *sir* gene into the pME1308 recombinant plasmid described in this patent application. This plasmid was named pME1325.

Overexpression of *metY* of *Wolinella succinogenes*

The *metY* gene as set forth in SEQ ID NO : 11, encoding the protein METY as set forth in SEQ ID NO : 12) of *Wolinella succinogenes* (ATCC29543D-5) was overexpressed by using the promoter of *E. coli metB* gene (Kirby *et al.,* 1986), the endogenous ribosome binding site of *metY gene* and the bacterial artificial chromosome (pCC1BAC, Epicentre). To optimize the overexpression, the star codon GTG of *metY was* also changed in ATG as set forth in SEQ ID NO : 13, thus encoding a methionine as the first amino acid instead of a valine as set forth in SEQ ID NO : 14). This plasmid was named pME1306.

Construction of strain 3: Overproduction of the protein thiocarboxylate-dependent methionine biosynthesis pathway of *Wolinella succinogenes* in a recombinant L-methionine producing *E. coli* strain

The plasmids pME1325 and pME1306 were transformed into strain 2, giving rise to the strain 3.

**EXAMPLE 2: Overproduction of MetX and MetY in a recombinant L-methionine producing *E. coli* strain deleted for *metA,* and without the alternative methionine biosynthesis pathway from *Wolinella succinogenes* - Construction of strain 4.**

The operon *hcySFD-sir* carried by the plasmid pME1325 was removed by using appropriate restriction enzymes. The resulting plasmid was named pME1338.

The plasmids pME1338 and pME1306 were transformed into strain 2, giving rise to the strain 4.

**EXAMPLE 3: Growth and L-methionine production with the alternative methionine biosynthesis pathway from *Wolinella succinogenes***

Production strains were evaluated in small Erlenmeyer flasks. A 5.5 mL preculture was grown at 37°C in a mixed medium (10 % LB medium (Sigma 25 %) with 2.5 $g.L^{-1}$ glucose and 90 % minimal medium PC1). It was used to inoculate a 50 mL culture to an $OD_{600}$ of 0.2 in medium PC1. Spectinomycin and kanamycin were added at a concentration of 50 $mg.L^{-1}$ and ampicillin at 10 $mg.L^{-1}$ when it was necessary. The temperature of the cultures was 37°C. When the culture had reached an $OD_{600}$ of 5 to 7, extracellular amino acids were quantified by HPLC after OPA/Fmoc derivatization and other relevant metabolites were analyzed using HPLC with refractometric detection (organic acids and glucose) and GC-MS after silylation.

The methionine titer was expressed as followed:

$$Titer = \frac{methionine\ (mol)}{volume\ (L)}$$

Table 1.: Minimal medium composition (PC1).

| Compound | Concentration ($g.L^{-1}$) |
|---|---|
| $ZnSO_4.7H_2O$ | 0.0040 |
| $CuCl_2.2H_2O$ | 0.0020 |
| $MnSO_4.H_2O$ | 0.0200 |
| $COCl_2.6H_2O$ | 0.0080 |

(continued)

| Compound | Concentration ($g.L^{-1}$) |
|---|---|
| $H_3BO_3$ | 0.0010 |
| $Na_2MoO_4.2H_2O$ | 0.0004 |
| $MgSO_4.7H_2O$ | 1.00 |
| Citric acid | 6.00 |
| $CaCl_2.2H_2O$ | 0.04 |
| $K_2HPO_4$ | 8.00 |
| $Na_2HPO_4$ | 2.00 |
| $(NH_4)_2HPO_4$ | 8.00 |
| $NH_4Cl$ | 0.13 |
| NaOH 4M | Adjusted to pH 6.8 |
| $FeSO_4.7H_2O$ | 0.04 |
| Thiamine | 0.01 |
| Glucose | 20.00 |
| Ammonium thiosulfate | 5.61 |
| Vitamin B12 | 0.01 |
| MOPS | 20.00 |
| IPTG | 0.0048 |

Table 2: Growth and L-methionine production levels (mM) for each strain tested in Erlenmeyer flasks in minimal medium. (-) means no growth at all (0 $h^{-1}$); (+) corresponds to a growth rate between 0 and 0.2 $h^{-1}$; (++) corresponds to a growth rate above 0.2 $h^{-1}$

| Strain | Growth rate | Methionine production (mM) |
|---|---|---|
| Strain 1 | + | 1.7 |
| Strain 2 | - | 0 |
| Strain 4 | + | 1.1 |
| Strain 3 | ++ | 1.8 |

The overexpression of the sulfur pathway from *W. succinogenes* in a recombinant L-methionine producing *E. coli* strain improves the growth rate and the L-methionine production (strain 3 compared to strain 4). This result shows that the alternative sulfur pathway functions in *E. coli* and improves the production of L-methionine. In strains 2, 3 and 4, the *metA*11* gene was deleted to demonstrate the functionality of the protein thiocarboxylate dependent methionine biosynthetic pathway. However, equivalent strains with a functional copy of *metA*11* gene on the chromosome were also constructed and evaluated in the same conditions. The results show that the metabolic pathway functions also in *E. coli metA*11* strains (data not shown).

All this results demonstrate that the alternative sulfur pathway from *Wolinella succinogenes* functions in *E. coli* and improves the production of the sulfur containing amino acid L-methionine. Moreover the use of such pathway overcomes problems linked to cysteine overproduction encountered usually for this production.

**REFERENCES**

- Agren D., Schnell R., Oehlmann W., Singh M., Schneider G., 2008, Journal of Biological Chemistry, 283(46):31567-31574.
- Altschul S, Gish W, Miller W, Myers E, Lipman DJ, 1990, J. Mol. Biol, 215 (3): 403-410.
- Anderson, 1946, Proc. Natl. Acad. Sci. USA., 32:120-128.
- Bourhy P, Martel A, Margarita D, Saint-Girons I and Belfaiza J. 1997, Journal of Bacteriology, 179 (13): 4396-4398.
- Carrier T & Keasling J. 1999, Biotechnol Prog., 15(1): 58-64.
- Datsenko K.A., Wanner B.L., 2000, Proceedings of the National Academy of Sciences of the USA, 97:6640-6645
- Davis JJ & Olsen GJ., 2011, Mol. Biol. Evol., 28(1):211-221.
- Deml L, Bojak A, Steck S, Graf M, Wild J, Schirmbeck R, Wolf H, Wagner R., 2001, J. Virol., 75(22): 10991-11001.
- Graf M, Bojak A, Deml L, Bieler K, Wolf H, Wagner R., 2000, J. Virol, 74(22): 10/22-10826.
- Kirby TW., Hindenach BR., Greene RC., 1986, Journal of Bacteriology, 165(3):671-677
- Krishnamoorthy K., Begley TP., 2011, Journal of the American Chemical Society, 133(2):379-386
- Lerner CG, Inouye M., 1990, Nucleic Acids Res. 1990 Aug 11;18(15):4631.
- Liebl W, Klamer R, Schleifer KH 1989, Appl. Microbiol. Biotechnol. 32: 205-210.
- Miller, 1992 "A Short Course in Bacterial Genetics: A Laboratory Manual and Handbook for Escherichia coli and Related Bacteria", Cold Spring Harbor Laboratory Press, Cold Spring Harbor, New York.
- Riedel C, Rittmann D, Dangel P, Möckel B, Petersen S, Sahm H, Eikmanns BJ., 2001, J. Mol. Microbiol. Biotechnol. 3: 573-583.
- Salis H, 2011, Methods Enzymol., 498:19-42.
- Saunderson C.L., 1985, British Journal of Nutrition, 54:621-633
- Schaefer U, Boos W, Takors R, Weuster-Botz D, 1999 Anal. Biochem. 270: 88-96.
- Segel IH, 1993, Enzyme kinetics, John Wiley & Sons, pp. 44-54 and 100-112.
- Taylor SV., Kelleher NL., Kinsland C., Chiu HJ., Costello CA., Backstrom AD., McLafferty FW., Begley TP., 1998, Journal of Biological Chemistry, 273(26):16555-16560.

## Claims

1. A genetically modified microorganism producing methionine by fermentation, wherein said microorganism is further genetically modified for

    - overexpressing the heterologous functional genes coding for the protein thiocarboxylate dependent methionine biosynthetic pathway of *Wolinella succinogenes, hcyS, hcyD, hcyF* and *sir* from *Wolinella succinogenes,* and for expressing functional genes encoding
    - a polypeptide having an homoserine O-acetyltransferase activity without feedback inhibition by methionine and/or S-adenosylmethionine and,
    - a polypeptide having O-acetylhomoserine sulfhydrylase activity.

2. The microorganism of claim 1, wherein the gene encoding a polypeptide having homoserine O-acetyltransferase activity without feedback inhibition by methionine and/or S-adenosylmethionine and the gene encoding a polypeptide having O-acetylhomoserine sulfhydrylase activity are heterologous.

3. The microorganism of claim 2, wherein the gene encoding a polypeptide having homoserine O-acetyltransferase activity without feedback inhibition by methionine and/or S-adenosylmethionine is *metX* gene from *Leptospira meyeri.*

4. The microorganism of claim 3 wherein *metX* gene from *Leptospira meyeri* is overexpressed.

5. The microorganism of claim 2, wherein the gene encoding a polypeptide having O-acetylhomoserine sulfhydrylase activity is *metY* gene from *Wolinella succinogenes.*

6. The microorganism of claim 5 wherein *metY* gene from *Wolinella succinogenes* is overexpressed.

7. The microorganism of anyone of claims 1 to 6 is further genetically modified for overexpressing at least one of the following genes: *thrA* or *thrA* allele encoding a polypeptide having aspartokinase/homoserine dehydrogenase activity with reduced feedback inhibition to threonine (*thrA**), *metL* encoding a polypeptide having bifunctional aspartokinase/homoserine dehydrogenase, *metE* encoding a polypeptide having cobalamin-independent methionine synthase or *metH* encoding a polypeptide having cobalamin-dependent methionine synthase.

8. The microorganism according to anyone of the preceding claims wherein it comprises the following genetic modifications:

    a. Increased expression of at least one the following genes: *pyc, ptsG, pntAB, cysP, cysU, cysW, cysA, cysM, cysJ, cysI, cysH, gcvT, gcvH, gcvP, lpd, glyA, serA, serB, serC, metF, fldA, fpr, metN, metI, metQ,* and/or
    b. Attenuated expression of at least one of the following genes: *metJ, pykA, pykF, purU, yncA, metE, dgsA, sgrS, sgrT, ygaZH* or *udhA.*

9. The microorganism according to anyone of claims 1 to 8, wherein said microorganism belongs to the family of *Enterobacteriaceae* or *Corynebacteriaceae.*

10. The microorganism according to claim 9, wherein said *Enterobacteriaceae* bacterium is *Escherichia coli.*

11. A method for the fermentative production of methionine comprising

    i. Culturing a genetically modified microorganism producing methionine overexpressing the heterologous functional genes coding for the protein thiocarboxylate dependent methionine biosynthetic pathway of *Wolinella succinogenes, hcyS, hcyD, hcyF* and *sir* from *Wolinella succinogenes* and expressing functional genes encoding a polypeptide having an homoserine O-acetyltransferase activity without feedback inhibition by methionine and/or S-adenosylmethionine and a polypeptide having O-acetylhomoserine sulfhydrylase activity and,
    ii. Recovering methionine from said culture medium.

12. The method of claim 11 wherein said genetically modified microorganism overexpresses the *metX* gene from *Leptospira meyeri* and the *metY* gene from *Wolinella succinogenes.*

13. The method of claim 11 or 12 wherein the genetically modified microorganism further overexpresses at least one of the following genes: *thrA* or *thrA* allele encoding a polypeptide having aspartokinase/homoserine dehydrogenase activity with reduced feedback inhibition to threonine (*thrA**), *metL* encoding a bifunctional aspartokinase/homoserine dehydrogenase, *metE* encoding a cobalamin-independent methionine synthase or *metH* encoding a cobalamin-dependent methionine synthase.

**Patentansprüche**

**1.** Genetisch modifizierter Mikroorganismus, der Methionin durch Fermentation produziert, wobei der Mikroorganismus ferner genetisch modifiziert ist zum:

- Überexprimieren der heterologen funktionellen Gene, kodierend für den Proteinthiocarboxylat-abhängigen Methionin-Biosyntheseweg von *Wolinella succinogenes, hcyS, hcyD, hcyF* und *sir* aus *Wolinella succinogenes,* und zum Exprimieren von funktionellen Genen, kodierend für
- ein Polypeptid mit einer Homoserin-O-Acetyltransferase-Aktivität ohne Rückkopplungshemmung durch Methionin und/oder S-Adenosylmethionin und
- ein Polypeptid mit O-Acetylhomoserin-Sulfhydrylase-Aktivität.

**2.** Mikroorganismus nach Anspruch 1, wobei das Gen, das ein Polypeptid mit Homoserin-O-Acetyltransferase-Aktivität ohne Rückkopplungshemmung durch Methionin und/oder S-Adenosylmethionin kodiert, und das Gen, das ein Polypeptid mit O-Acetylhomoserin-Sulfhydrylase-Aktivität kodiert, heterolog sind.

**3.** Mikroorganismus nach Anspruch 2, wobei das Gen, das für ein Polypeptid mit Homoserin-O-Acetyltransferase-Aktivität ohne Rückkopplungshemmung durch Methionin und/oder S-Adenosylmethionin kodiert, ein metX-Gen aus *Leptospira meyeri* ist.

**4.** Mikroorganismus nach Anspruch 3, wobei das *metX*-Gen aus *Leptospira meyeri* überexprimiert wird.

**5.** Mikroorganismus nach Anspruch 2, wobei das Gen, das für ein Polypeptid mit O-Acetylhomoserin-Sulfhydrylase-Aktivität kodiert, das metY-Gen aus *Wolinella succinogenes* ist.

**6.** Mikroorganismus nach Anspruch 5, wobei das *metY*-Gen aus *Wolinella succinogenes* überexprimiert wird.

**7.** Mikroorganismus nach einem der Ansprüche 1 bis 6, der ferner genetisch modifiziert ist zum Überexprimieren mindestens eines der folgenden Gene: thrA oder thrA-Allel, kodierend ein Polypeptid mit Aspartokinase/Homoserin-Ddehydrogenase-Aktivität mit verringerter Rückkopplungshemmung gegenüber Threonin (*thrA**), *metL,* kodierend ein Polypeptid von bifunktioneller Aspartokinase/Homoserin-Dehydrogenase, *metE,* kodierend ein Polypeptid von Cobalamin-unabhängiger Methionin-Synthase, oder *metH,* kodierend ein Polypeptid von Cobalamin-abhängiger Methioninsynthase.

**8.** Mikroorganismus nach einem der vorhergehenden Ansprüche, wobei dieser die folgenden genetischen Modifikationen umfasst:

a. erhöhte Expression von mindestens einem der folgenden Gene: *pyc, ptsG, pntAB, cysP, cysU, cysW*, *cysA, cysM, cysJ, cysI, cysH, gcvT, gcvH, gcvP, Ipd, glyA, serA, serB, serC, metF, fldA, fpr, metN, metI, met*Q und/oder
b. abgeschwächte Expression von mindestens einem der folgenden Gene: *metJ, pykA, pykF, purU, yncA, metE, dgsA, sgrS, sgrT, ygaZH* oder *udhA.*

**9.** Mikroorganismus nach einem der Ansprüche 1 bis 8, wobei besagter Mikroorganismus zur Familie der *Enterobacteriaceae* oder *Corynebacteriaceae* gehört.

**10.** Mikroorganismus nach Anspruch 9, wobei es sich bei besagtem *Enterobacteriaceae-Bakterium* um *Escherichia coli* handelt.

**11.** Verfahren zur fermentativen Produktion von Methionin, umfassend

i. Kultivieren eines Methionin produzierenden, genetisch modifizierten Mikroorganismus, der die heterologen funktionellen Gene überexprimiert, kodierend für den Proteinthiocarboxylat-abhängigen Methionin-Biosyntheseweg von *Wolinella succinogenes, hcyS, hcyD, hcyF* und *sir* aus *Wolinella succinogenes,* und funktionelle Gene exprimiert, kodierend für ein Polypeptid mit einer Homoserin-O-Acetyltransferase-Aktivität ohne Rückkopplungshemmung durch Methionin und/oder S-Adenosylmethionin und für ein Polypeptid mit O-Acetylhomoserin-Sulfhydrylase-Aktivität, und
ii. Rückgewinnen von Methionin aus dem Kulturmedium.

**12.** Verfahren nach Anspruch 11, wobei der genetisch modifizierte Mikroorganismus das metX-Gen aus *Leptospira meyeri* und das metY-Gen aus *Wolinella succinogenes* überexprimiert.

**13.** Verfahren nach Anspruch 11 oder 12, wobei der genetisch modifizierte Mikroorganismus ferner mindestens eines der folgenden Gene überexprimiert:
*thrA* oder *thrA*-Allel, kodierend ein Polypeptid mit Aspartokinase/Homoserin-Ddehydrogenase-Aktivität mit verringerter Rückkopplungshemmung gegenüber Threonin (*thrA**), *metL,* kodierend eine bifunktionelle Aspartokinase/Homoserin-Dehydrogenase, *metE,* kodierend eine Cobalamin-unabhängige Methionin-Synthase, oder *metH,* kodierend eine Cobalamin-abhängige Methioninsynthase.

## Revendications

**1.** Microorganisme génétiquement modifié produisant de la méthionine par fermentation, ledit microorganisme étant en outre génétiquement modifié pour

- surexprimer les gènes fonctionnels hétérologues codant pour la voie biosynthétique de la méthionine, dépendante de la protéine-thiocarboxylate, de *Wolinella succinogenes, hcyS, hcyD, hcyF* et *sir* issus de *Wolinella succinogenes,*
et pour exprimer des gènes fonctionnels codant pour
- un polypeptide ayant une activité d'homosérine 0-acétyltransférase sans rétro-inhibition par la méthionine et/ou la S-adénosylméthionine ; et
- un polypeptide ayant une activité d'O-acétylhomosérine sulfhydrylase.

**2.** Microorganisme selon la revendication 1, dans lequel le gène codant pour un polypeptide ayant une activité d'homosérine O-acétyltransférase sans rétro-inhibition par la méthionine et/ou la S-adénosylméthionine et le gène codant pour un polypeptide ayant une activité d'O-acétylhomosérine sulfhydrylase, sont hétérologues.

**3.** Microorganisme selon la revendication 2, dans lequel le gène codant pour un polypeptide ayant une activité d'homosérine O-acétyltransférase sans rétro-inhibition par la méthionine et/ou la S-adénosylméthionine est le gène *metX* issu de *Leptospira meyeri.*

**4.** Microorganisme selon la revendication 3, dans lequel le gène *metX* issu de *Leptospira meyeri* est surexprimé.

**5.** Microorganisme selon la revendication 2, dans lequel le gène codant pour un polypeptide ayant une activité d'O-acétylhomosérine sulfhydrylase est le gène *metY* issu de *Wolinella succinogenes.*

**6.** Microorganisme selon la revendication 5, dans lequel le gène *metY* issu de *Wolinella succinogenes* est surexprimé.

**7.** Microorganisme selon l'une quelconque des revendications 1 à 6, qui est en outre génétiquement modifié pour surexprimer au moins l'un parmi les gènes suivants : *thrA* ou un allèle *thrA* codant pour un polypeptide ayant une activité d'aspartate kinase/homosérine déshydrogénase ayant une rétro-inhibition réduite vis-à-vis de la thréonine (*thrA**), *metL* codant pour un polypeptide ayant une aspartate kinase/homosérine déshydrogénase bifonctionnelle, *metE* codant pour un polypeptide ayant une méthionine synthase cobalamine-indépendante ou *metH* codant pour un polypeptide ayant une méthionine synthase cobalamine-dépendante.

**8.** Microorganisme selon l'une quelconque des revendications précédentes, comprenant les modifications génétiques suivantes :

a. une expression accrue d'au moins l'un parmi les gènes suivants : *pyc, ptsG, pntAB, cysP, cysU, cysW, cysA, cysM, cysJ, cysI, cysH, gcvT, gcvH, gcvP, Ipd, glyA, serA, serB, serC, metF, fldA, fpr, metN, metI, metQ,* et/ou
b. une expression atténuée d'au moins l'un parmi les gènes suivants : *metJ, pykA, pykF, purU, yncA, metE,*

*dgsA, sgrS, sgrT, ygaZH* ou *udhA.*

**9.** Microorganisme selon l'une quelconque des revendications 1 à 8, ledit microorganisme appartenant à la famille des *Enterobacteriaceae* ou *Corynebacteriaceae.*

**10.** Microorganisme selon la revendication 9, dans lequel ladite bactérie de type *Enterobacteriaceae* est *Escherichia coli.*

**11.** Méthode de production fermentative de méthionine, comprenant :

i. la culture d'un microorganisme génétiquement modifié produisant de la méthionine, surexprimant les gènes fonctionnels hétérologues codant pour la voie biosynthétique de la méthionine, dépendante de la protéine-thiocarboxylate, de *Wolinella succinogenes, hcyS, hcyD, hcyF* et *sir* issus de *Wolinella succinogenes,* et exprimant les gènes fonctionnels codant pour un polypeptide ayant une activité d'homosérine 0-acétyltransférase sans rétro-inhibition par la méthionine et/ou la S-adénosylméthionine et un polypeptide ayant une activité d'O-acétylhomosérine sulfhydrylase ; et
ii. la récupération de méthionine à partir dudit milieu de culture.

**12.** Méthode selon la revendication 11, dans laquelle ledit microorganisme génétiquement modifié surexprime le gène *metX* issu de *Leptospira meyeri* et le gène *metY* issu de *Wolinella succinogenes.*

**13.** Méthode selon la revendication 11 ou 12, dans laquelle le microorganisme génétiquement modifié surexprime en outre au moins l'un parmi les gènes suivants : *thrA* ou un allèle *thrA* codant pour un polypeptide ayant une activité d'aspartate kinase/homosérine déshydrogénase ayant une rétro-inhibition réduite vis-à-vis de la thréonine (*thrA**), *metL* codant pour une aspartate kinase/homosérine déshydrogénase bifonctionnelle, *metE* codant pour une méthionine synthase cobalamine-indépendante ou *metH* codant pour une méthionine synthase cobalamine-dépendante.

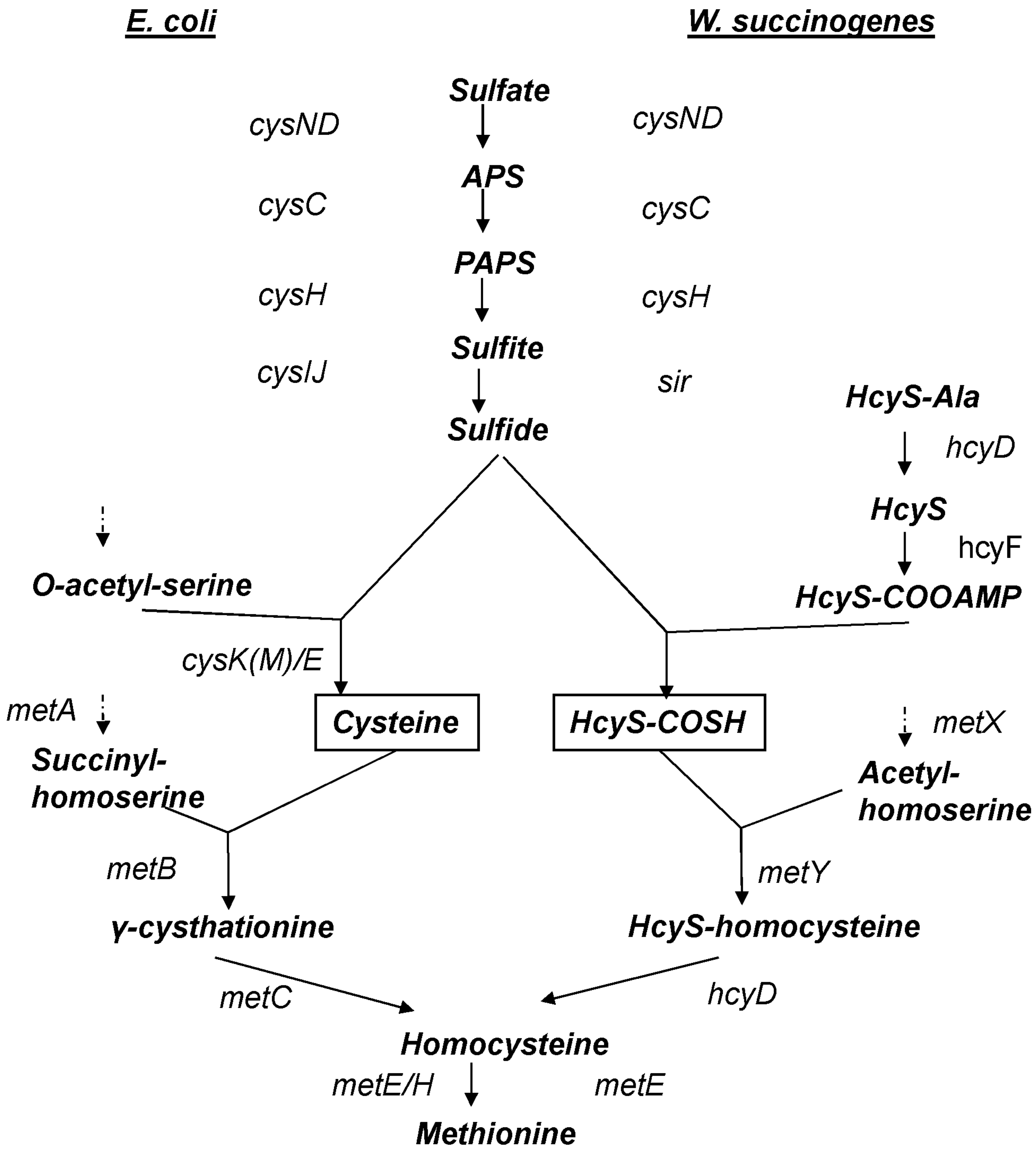
E. coli
W. succinogenes
Sulfate
cysND
cysND
APS
cysC
cysC
PAPS
cysH
cysH
Sulfite
cysIJ
sir
Sulfide
HcyS-Ala
hcyD
HcyS
hcyF
HcyS-COOAMP
O-acetyl-serine
cysK(M)/E
Cysteine
HcyS-COSH
metA
metX
Succinyl-homoserine
Acetyl-homoserine
metB
metY
γ-cysthationine
HcyS-homocysteine
metC
hcyD
Homocysteine
metE/H
metE
Methionine

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7790424 B
- US 7611873 B
- WO 0210209 A
- WO 2005059093 A
- WO 2006008097 A
- WO 20070770441 A
- WO 2009043803 A
- WO 2012098042 A
- WO 2005111202 A
- WO 2008013432 A
- WO 2008127240 A
- WO 2005073364 A
- WO 2008116852 A
- WO 2007077041 A
- WO 2010020681 A
- WO 2011073738 A
- WO 2011080542 A
- WO 2011080301 A
- WO 2012055798 A
- WO 2013001055 A
- WO 2013190343 A
- WO 2015028675 A
- WO 2015028674 A
- US 8551742 B
- EP 2290051 A
- WO 200707704 A
- JP 2000157267 A
- WO 2011073122 A
- WO 2005007862 A
- WO 2005059155 A


### Non-patent literature cited in the description


- **ANDERSON.** *Proc. Natl. Acad. Sci. USA,* 1946, vol. 32, 120-128
- **MILLER.** A Short Course in Bacterial Genetics: A Laboratory Manual and Handbook for Escherichia coli and Related Bacteria. Cold Spring Harbor Laboratory Press, 1992
- **SCHAEFER et al.** *Biochem.,* 1999, vol. 270, 88-96
- **LIEBL et al.** *Appl. Microbiol. Biotechnol.,* 1989, vol. 32, 205-210
- **RIEDEL et al.** *J. Mol. Microbiol. Biotechnol.,* 2001, vol. 3, 573-583
- **AGREN D. ; SCHNELL R. ; OEHLMANN W. ; SINGH M. ; SCHNEIDER G.** *Journal of Biological Chemistry,* 2008, vol. 283 (46), 31567-31574
- **ALTSCHUL S ; GISH W ; MILLER W ; MYERS E ; LIPMAN DJ.** *J. Mol. Biol,* 1990, vol. 215 (3), 403-410
- **ANDERSON.** *Proc. Natl. Acad. Sci. USA.,* 1946, vol. 32, 120-128
- **BOURHY P ; MARTEL A ; MARGARITA D ; SAINT-GIRONS I ; BELFAIZA J.** *Journal of Bacteriology,* 1997, vol. 179 (13), 4396-4398
- **CARRIER T ; KEASLING J.** *Biotechnol Prog.,* 1999, vol. 15 (1), 58-64
- **DATSENKO K.A. ; WANNER B.L.** *Proceedings of the National Academy of Sciences of the USA,* 2000, vol. 97, 6640-6645
- **DAVIS JJ ; OLSEN GJ.** *Mol. Biol. Evol.,* 2011, vol. 28 (1), 211-221
- **DEML L ; BOJAK A ; STECK S ; GRAF M ; WILD J ; SCHIRMBECK R ; WOLF H ; WAGNER R.** *J. Virol.,* 2001, vol. 75 (22), 10991-11001
- **GRAF M ; BOJAK A ; DEML L ; BIELER K ; WOLF H ; WAGNER R.** *J. Virol,* 2000, vol. 74 (22), 10, , 22-10826
- **KIRBY TW. ; HINDENACH BR. ; GREENE RC.** *Journal of Bacteriology,* 1986, vol. 165 (3), 671-677
- **KRISHNAMOORTHY K. ; BEGLEY TP.** *Journal of the American Chemical Society,* 2011, vol. 133 (2), 379-386
- **LERNER CG ; INOUYE M.** *Nucleic Acids Res.,* 11 August 1990, vol. 18 (15), 4631
- **LIEBL W ; KLAMER R ; SCHLEIFER KH.** *Appl. Microbiol. Biotechnol.,* 1989, vol. 32, 205-210
- **RIEDEL C ; RITTMANN D ; DANGEL P ; MÖCKEL B ; PETERSEN S ; SAHM H ; EIKMANNS BJ.** *J. Mol. Microbiol. Biotechnol.,* 2001, vol. 3, 573-583
- **SALIS H.** *Methods Enzymol.,* 2011, vol. 498, 19-42
- **SAUNDERSON C.L.** *British Journal of Nutrition,* 1985, vol. 54, 621-633
- **SCHAEFER U ; BOOS W ; TAKORS R ; WEUSTER-BOTZ D.** *Anal. Biochem.,* 1999, vol. 270, 88-96

- **SEGEL IH.** Enzyme kinetics. John Wiley & Sons, 1993, 44-54, 100-112
- **TAYLOR SV. ; KELLEHER NL. ; KINSLAND C. ; CHIU HJ. ; COSTELLO CA. ; BACKSTROM AD. ; MCLAFFERTY FW. ; BEGLEY TP.** *Journal of Biological Chemistry,* 1998, vol. 273 (26), 16555-16560